# EUROPEAN PATENT SPECIFICATION

(11) **EP 4 520 391 B1**
(45) Date of publication and mention of the grant of the patent: **12.08.2026**
(21) Application number: 25154364.1
(22) Date of filing: 17.06.2022
(51) Int. Cl.: A61B 5/00, A61N 1/36

(54) **LATENCY COMPENSATION FOR DETECTION OF ECAPS**
LATENZKOMPENSATION ZUR DETEKTION VON EIPS
COMPENSATION DE LATENCE POUR LA DÉTECTION D'ECAPS

(30) Priority: 17.06.2021 US 202163211867 P; 16.06.2022 US 202217807192
(43) Date of publication of application: 12.03.2025
(62) Document number(s) of the earlier application(s) in accordance with Art. 76 EPC: 22743988.2 / 4 355 201
(73) Proprietor: Medtronic, Inc., Minneapolis, Minnesota 55432 (US)
(72) Inventor: DINSMOOR, David A., Minneapolis, MN 55432 (US)
(74) Representative: Zimmermann & Partner Patentanwälte mbB

(56) References cited:
- WO-A1-2022/182827
- US-A1- 2016 175 594
- US-A1- 2020 179 688
- US-A1- 2021 121 700
- US-A1- 2021 138 250

## Description

This application claims priority to U.S. Patent Application. No. 17/807,192, filed June 16, 2022, and U.S. Provisional Patent Application No. 63/211,867, filed June 17, 2021.

### TECHNICAL FIELD

This disclosure generally relates to electrical stimulation, and more specifically, control of electrical stimulation.

### BACKGROUND

Medical devices may be external or implanted and may be used to deliver electrical stimulation therapy to patients via various tissue sites to treat a variety of symptoms or conditions such as chronic pain, tremor, Parkinson's disease, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Stimulation proximate the spinal cord, proximate the sacral nerve, within the brain, and proximate peripheral nerves are often referred to as spinal cord stimulation (SCS), sacral neuromodulation (SNM), deep brain stimulation (DBS), and peripheral nerve stimulation (PNS), respectively.

Electrical stimulation may be delivered to a patient by the medical device in a train of electrical pulses, and parameters of the electrical pulses may include a frequency, an amplitude, a pulse width, and a pulse shape. An evoked compound action potential (ECAP) is synchronous firing of a population of neurons which occurs in response to the application of a stimulus including, in some cases, an electrical stimulus by a medical device. The ECAP may be detectable as being a separate event from the stimulus itself, and the ECAP may reveal characteristics of the effect of the stimulus on the nerve fibers. Document WO 2022/182827 A1 is a post published document and relates to stimulation devices.

### SUMMARY

In general, systems, devices, and techniques are described for sensing evoked compound action potential (ECAP) signals from a patient. ECAP signals (which may be referred to in the plurality as ECAPS) are a measure of neural recruitment because each ECAP signal represents the superposition of electrical potentials generated from a population of axons firing in response to an electrical stimulus (e.g., a stimulation pulse). Changes in a feature or characteristic (e.g., an amplitude of a portion of the signal or area under the curve of the signal) of ECAP signals occur as a function of how many axons have been activated by the delivered stimulation pulse. A system can use the ECAP signals for a variety of purposes, such as a closed-loop feedback variable to inform electrical stimulation therapy adjustments. However, the latency (e.g., the delay) of one or more features of the ECAP signal elicited by the electrical stimulus can depend on one or more stimulation parameter values that define the electrical stimulus.

As described herein, a system may determine a sensing window for sensing one or more features of an ECAP signal based on a value of a stimulation parameter that at least partially defined the stimulation pulse that elicited the ECAP signal. For example, the latency of some or all features of ECAP signals may increase as the current amplitude increases for subsequent stimulation pulses. The system can then adjust one or more aspects of a sensing window (e.g., the window duration or the start of the sensing window after the stimulation pulse) that the system uses to detect one or more features of the ECAP signal based on the value of the stimulation parameter that defines the stimulation pulse that elicited the ECAP signal. The system may determine the relationship between the latency and the stimulation parameter values for a specific patient, as the relationship may be dependent on patient specific conditions such as electrode implant location, patient anatomy, disease state, neurological conditions, or any other patient-specific conditions that affect response to electrical stimulus.

In one example, a system includes processing circuitry configured to determine a value of a stimulation parameter that at least partially defines an electrical stimulation pulse; select, based on the value of the stimulation parameter, a sensing window for detecting one or more features of a sensed evoked compound action potential (ECAP) signal elicited by the electrical stimulation pulse; determine a value of each of the one or more features within the sensing window from the sensed ECAP signal; and control, based on the value of each of the one or more features, subsequent electrical stimulation deliverable to a patient.

In another example, a method includes determining, by processing circuitry, a value of a stimulation parameter that at least partially defines an electrical stimulation pulse; selecting, by the processing circuitry and based on the value of the stimulation parameter, a sensing window for detecting one or more features of a sensed evoked compound action potential (ECAP) signal elicited by the electrical stimulation pulse; determining, by the processing circuitry, a value of each of the one or more features within the sensing window from the sensed ECAP signal; and controlling, by the processing circuitry and based on the value of each of the one or more features, subsequent electrical stimulation deliverable to a patient.

In another example, a computer-readable storage medium comprising instructions that, when executed by processing circuitry, cause the processing circuitry to determine a value of a stimulation parameter that at least partially defines an electrical stimulation pulse; select, based on the value of the stimulation parameter, a sensing window for detecting one or more features of a sensed evoked compound action potential (ECAP) signal elicited by the electrical stimulation pulse; determine, a value of each of the one or more features within the sensing window from the sensed ECAP signal; and control, based on the value of each of the one or more features, subsequent electrical stimulation deliverable to a patient.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages of the techniques will be apparent from the description and drawings, and from the claims. The present invention is set out in the appended claims.

### BRIEF DESCRIPTION OF DRAWINGS

FIG. 1 is a conceptual diagram illustrating an example system that includes a medical device programmer and an IMD according to the techniques of the disclosure.
FIG. 2 is a block diagram of the example IMD of FIG. 1.
FIG. 3 is a block diagram of the example external programmer of FIG. 1.
FIG. 4A is a graph of an example ECAP signal sensed from a stimulation pulse.
FIGS. 4B and 4C are graphs of example ECAP signals sensed from stimulation pulses having different current amplitudes.
FIG. 5 is a timing diagram illustrating one example of electrical stimulation pulses and respective sensed ECAPs, in accordance with one or more techniques of this disclosure.
FIG. 6A is a graph of example growth curves derived from sensed ECAPs during respective posture states.
FIG. 6B is a graph of example latency curves derived from sensed ECAPs elicited by stimulation pulses having different parameter values.
FIG. 7A is a flow diagram illustrating an example technique for selecting a sensing window for sensing an ECAP signal based on a stimulation parameter of a stimulation pulse that elicits the ECAP signal, in accordance with one or more techniques of this disclosure.
FIG. 7B is a flow diagram illustrating an example technique for determining a relationship between latency of ECAP features and stimulation parameter values, in accordance with one or more techniques of this disclosure.
FIG. 8 is a diagram illustrating an example technique for adjusting electrical stimulation therapy.
FIG. 9 is a graph illustrating a relationship between sensed ECAP voltage amplitude and stimulation current amplitude, in accordance with one or more techniques of this disclosure.

Like reference characters denote like elements throughout the description and figures.

### DETAILED DESCRIPTION

The disclosure describes examples of medical devices, systems, and techniques for sensing ECAP signals that may be utilized for various diagnostic or therapeutic purposes. Electrical stimulation therapy is typically delivered to a target tissue (e.g., nerves of the spinal cord or muscle) of a patient via two or more electrodes. Parameters of the electrical stimulation therapy (e.g., electrode combination, voltage or current amplitude, pulse width, pulse frequency, etc.) are selected by a clinician and/or the patient to provide relief from various symptoms, such as pain, nervous system disorders, muscle disorders, etc. However, as the patient moves, the distance between the electrodes and the target tissues changes. Since neural recruitment at the nerves is a function of stimulation intensity (e.g., amplitude and/or pulse frequency) and distance between the target tissue and the electrodes, movement of the electrode closer to the target tissue may result in increased neural recruitment (e.g., possible painful sensations or adverse motor function), and movement of the electrode further from the target tissue may result in decreased efficacy of the therapy for the patient. Certain patient postures (which may or may not include patient activity) may be representative of respective distances (or changes in distance) between electrodes and nerves and thus be an informative feedback variable for modulating stimulation therapy.

In some examples, a patient may experience discomfort or pain caused by transient patient conditions, which is referred to herein as transient overstimulation. The electrodes can move closer to the target tissue for a number of reasons including coughing, sneezing, laughing, valsalva maneuvers, leg lifting, cervical motions, deep breathing, or another transient patient movement. If a system is delivering stimulation during these movements, the patient may perceive the stimulation as stronger (and possibly uncomfortable) due to the decreased distance between electrodes and target tissue in a short amount of time. Although a patient may anticipate such movements and preemptively reduce stimulation intensity in an attempt to avoid these uncomfortable sensations, these patient actions interfere with normal activities and may not be sufficient to avoid uncomfortable stimulation at all times.

ECAPs are a measure of neural recruitment because each ECAP signal represents the superposition of electrical potentials generated from a population of axons firing in response to an electrical stimulus (e.g., a stimulation pulse). Changes in a feature or characteristic (e.g., an amplitude of a portion of the signal or area under the curve of the signal) of ECAP signals occur as a function of how many axons have been activated by the delivered stimulation pulse. For a given set of parameter values that define the stimulation pulse and a given distance between the electrodes and target nerve, the detected ECAP signal may have a certain characteristic value (e.g., amplitude, or area under a curve). Therefore, a system can detect one or more features of a sensed ECAP signal and determine that the distance between electrodes and nerves has increased or decreased in response to determining that the feature (or measured ECAP characteristic value based on one or more features) has increased or decreased. For example, if the set of parameter values stays the same and the ECAP characteristic value of amplitude increases, the system can determine that the distance between electrodes and the nerve has decreased.

The ECAP signal may include several features such as different peaks that include a first peak (P1), a trough (N1), a second peak (P2), and sometimes additional troughs and peaks. The system may determine an ECAP characteristic value for the ECAP signal based on one or more of these features (e.g., the absolute amplitude between the N1 and P2 features). However, the detection of one or more features of the ECAP signal may be difficult as features of the ECAP signal may change in time from delivery of the stimulation pulse that elicits the ECAP signal. This change in the delay of the ECAP features from the delivered stimulation pulse can be referred to as latency. A change in latency of the features in the ECAP signal may be due to different nerve fibers being activated from differences in the parameter values of the stimulation pulse. A system may employ sensing windows to identify respective features in the ECAP signal, but if a change in latency causes a feature to occur outside for the sensing window, the system may not identify the appropriate feature of the ECAP signal. A consequence of failing to identify the appropriate feature in the ECAP signal may result in an inability of the system to appropriate adjust electrical stimulation therapy based on the sensed ECAP signals.

As described herein, a system may determine a sensing window for sensing one or more features of an ECAP signal based on a value of a stimulation parameter that at least partially defined the stimulation pulse that elicited the ECAP signal. For example, the latency of one or more (or all) features of ECAP signals may increase as the current amplitude increases for subsequent stimulation pulses. The system can then adjust, based on the value of the stimulation parameter that defines the stimulation pulse that elicited the ECAP signal, one or more aspects of a sensing window (e.g., the window duration or the start of the sensing window after the stimulation pulse) that the system uses to detect one or more features of the ECAP signal.

The relationship between stimulation pulse parameter values and latency of features in the ECAP signal may be patient-dependent. In other words, the system may select the sensing window to accommodate the specific way that the nerves of the patient react to delivered stimulus. Therefore, the system may determine the relationship between the latency and the stimulation parameter values for the specific patient, as the relationship may be dependent on patient specific conditions such as electrode implant location, patient anatomy, disease state, neurological conditions, or any other patient-specific conditions that affect response to electrical stimulus. Prior to delivery of therapy, or periodically over the course of therapy, the system may deliver a sweep of stimulation pulses with different parameter values and measure the latency between one or more features of the ECAP signal for each parameter value. The system can then generate the relationship between the parameter values and one or more aspects of the sensing window. The system then can select appropriate sensing windows for an ECAP signal based on the parameter values used to define the delivered stimulation pulse that elicits the ECAP signal. By selecting or adjusting the sensing window in this manner, the system may detect the appropriate features from the ECAP signal that the system uses to generate the ECAP characteristic used for closed-loop control of stimulation therapy.

Effective stimulation therapy may rely on a certain level of neural recruitment at a target nerve (e.g., at a target ECAP characteristic value or below a threshold ECAP characteristic value). This effective stimulation therapy may provide relief from one or more conditions (e.g., patient perceived pain) without an unacceptable level of side effects (e.g., overwhelming perception of stimulation). In order to maintain effective stimulation therapy, a system may use the characteristic value of an ECAP signal as feedback for adjusting a stimulation parameter (e.g., increase or decrease the stimulation parameter value) to increase or decrease the neural recruitment back to the neural recruitment associated with effective stimulation therapy. However, the patient may have different sensitivities to increasing stimulation intensity and decreasing stimulation intensity. For example, the patient may be more sensitive to increasing stimulation intensity (e.g., increasing a current amplitude value in a subsequent pulse) than decreasing stimulation intensity (e.g., decreasing a current amplitude value in a subsequent pulse). Without tailoring changes to stimulation parameter values to account for increasing or decreasing stimulation intensity of stimulation pulses, the system may not efficiently achieve the desired neural recruitment levels for the patient (e.g., cause patient discomfort or reduce therapy efficacy). Therefore, a system may employ different gain values for increasing stimulation intensity and decreasing stimulation intensity, as determined by the difference between a target ECAP characteristic value and the detected ECAP characteristic value (e.g., an ECAP differential value). Again, the system may select the appropriate sensing window based on stimulation pulse parameter values in order to detect appropriate features of the ECAP signal and generate the ECAP characteristic value.

Moreover, if the patient changes posture or otherwise engages in physical activity, the distance between the electrodes and the nerve changes as well. This change in distance can cause loss of effective therapy and/or side effects if the parameter values that define stimulation are not adjusted to compensate for the change in distance. The different distance between electrodes and the target nerve (e.g., caused by a shift from one posture state to another) may also result in different sensitivities to stimulation intensity (e.g., smaller distances may result in greater sensitivities to changes in stimulation intensity). If a system does not adjust the control policy for these changes, adjustments to stimulation parameter values may not be sufficient to maintain effective therapy or may provide stimulation that is too strong at that posture state. Therefore, it may be beneficial to maintain effective therapy by the system adjusting how stimulation intensity is changed within a given posture state and/or changing target ECAP characteristic values when a posture state of the patient has changed. A system may thus employ a closed-loop control system for adjusting parameter values of electrical stimulation pulses based on the features of sensed ECAP signals.

Although electrical stimulation is generally described herein in the form of electrical stimulation pulses, electrical stimulation may be delivered in non-pulse form in other examples. For example, electrical stimulation may be delivered as a signal having various waveform shapes, frequencies, and amplitudes. Therefore, electrical stimulation in the form of a non-pulse signal may be a continuous signal than may have a sinusoidal waveform or other continuous waveform.

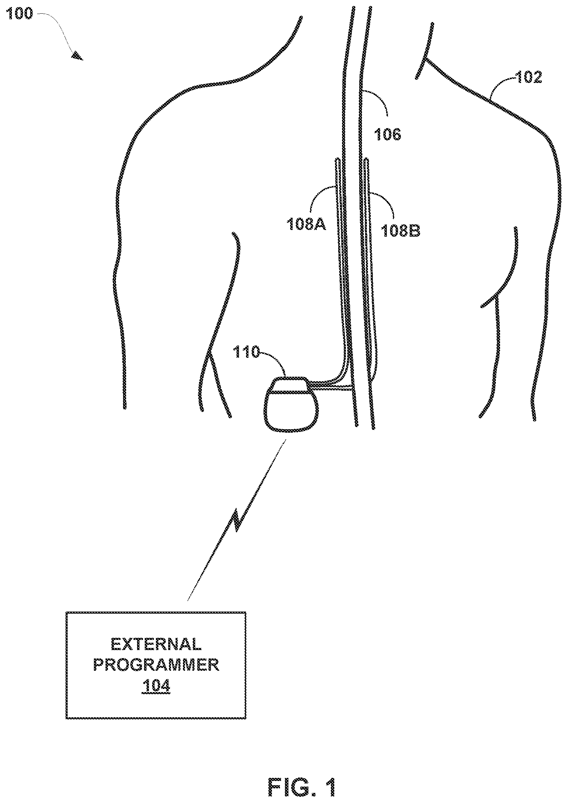

FIG. 1 is a conceptual diagram illustrating example system 100 that includes implantable medical device (IMD) 110 to deliver electrical stimulation therapy to patient 102. Although the techniques described in this disclosure are generally applicable to a variety of medical devices including external devices and IMDs, application of such techniques to IMDs and, more particularly, implantable electrical stimulators (e.g., neurostimulators) will be described for purposes of illustration. More particularly, the disclosure will refer to an implantable SCS system for purposes of illustration, but without limitation as to other types of medical devices or other therapeutic applications of medical devices.

As shown in FIG. 1, system 100 includes an IMD 110, leads 108A and 108B, and external programmer 104 shown in conjunction with a patient 102, who is ordinarily a human patient. In the example of FIG. 1, IMD 110 is an implantable electrical stimulator that is configured to generate and deliver electrical stimulation therapy to patient 102 via one or more electrodes of electrodes of leads 108A and/or 108B (collectively, "leads 108"), e.g., for relief of chronic pain or other symptoms. In other examples, IMD 110 may be coupled to a single lead carrying multiple electrodes or more than two leads each carrying multiple electrodes. In some examples, the stimulation signals, or pulses, may be configured to elicit detectable ECAP signals that IMD 110 may use to determine the posture state occupied by patient 102 and/or determine how to adjust one or more parameters that define stimulation therapy. IMD 110 may be a chronic electrical stimulator that remains implanted within patient 102 for weeks, months, or even years. In other examples, IMD 110 may be a temporary, or trial, stimulator used to screen or evaluate the efficacy of electrical stimulation for chronic therapy. In one example, IMD 110 is implanted within patient 102, while in another example, IMD 110 is an external device coupled to percutaneously implanted leads. In some examples, IMD 110 uses one or more leads, while in other examples, IMD 110 is leadless.

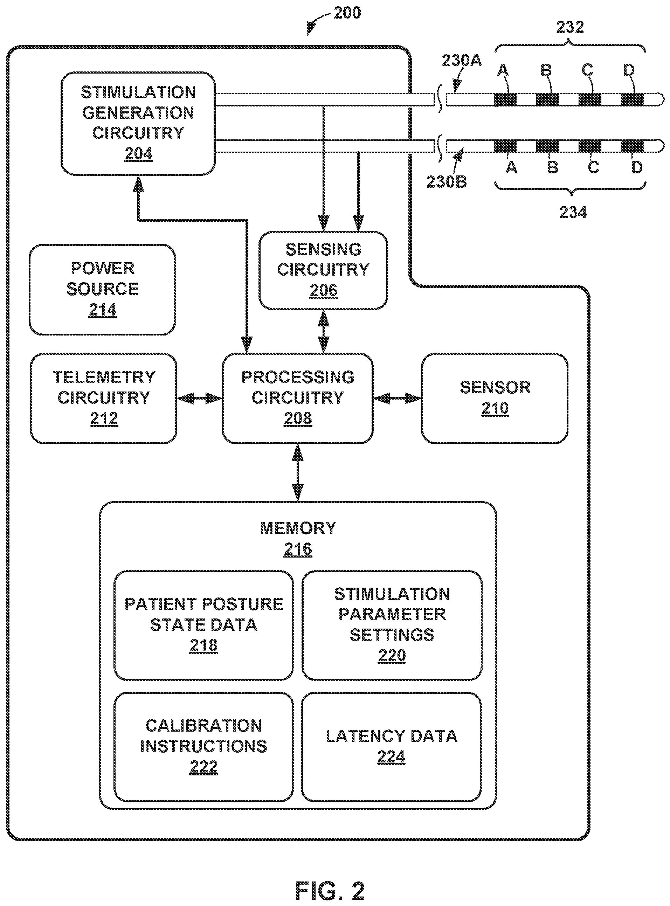

IMD 110 may be constructed of any polymer, metal, or composite material sufficient to house the components of IMD 110 (e.g., components illustrated in FIG. 2) within patient 102. In this example, IMD 110 may be constructed with a biocompatible housing, such as titanium or stainless steel, or a polymeric material such as silicone, polyurethane, or a liquid crystal polymer, and surgically implanted at a site in patient 102 near the pelvis, abdomen, or buttocks. In other examples, IMD 110 may be implanted within other suitable sites within patient 102, which may depend, for example, on the target site within patient 102 for the delivery of electrical stimulation therapy. The outer housing of IMD 110 may be configured to provide a hermetic seal for components, such as a rechargeable or non-rechargeable power source. In addition, in some examples, the outer housing of IMD 110 is selected from a material that facilitates receiving energy to charge the rechargeable power source.

Electrical stimulation energy, which may be constant current or constant voltage-based pulses, for example, is delivered from IMD 110 to one or more target tissue sites of patient 102 via one or more electrodes (not shown) of implantable leads 108. In the example of FIG. 1, leads 108 carry electrodes that are placed adjacent to the target tissue of spinal cord 106. One or more of the electrodes may be disposed at a distal tip of a lead 108 and/or at other positions at intermediate points along the lead. Leads 108 may be implanted and coupled to IMD 110. The electrodes may transfer electrical stimulation generated by an electrical stimulation generator in IMD 110 to tissue of patient 102. Although leads 108 may each be a single lead, lead 108 may include a lead extension or other segments that may aid in implantation or positioning of lead 108. In some other examples, IMD 110 may be a leadless stimulator with one or more arrays of electrodes arranged on a housing of the stimulator rather than leads that extend from the housing. In addition, in some other examples, system 100 may include one lead or more than two leads, each coupled to IMD 110 and directed to similar or different target tissue sites.

The electrodes of leads 108 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes) or any other type of electrodes capable of forming unipolar, bipolar or multipolar electrode combinations for therapy. Ring electrodes arranged at different axial positions at the distal ends of lead 108 will be described for purposes of illustration.

The deployment of electrodes via leads 108 is described for purposes of illustration, but arrays of electrodes may be deployed in different ways. For example, a housing associated with a leadless stimulator may carry arrays of electrodes, e.g., rows and/or columns (or other patterns), to which shifting operations may be applied. Such electrodes may be arranged as surface electrodes, ring electrodes, or protrusions. As a further alternative, electrode arrays may be formed by rows and/or columns of electrodes on one or more paddle leads. In some examples, electrode arrays include electrode segments, which are arranged at respective positions around a periphery of a lead, e.g., arranged in the form of one or more segmented rings around a circumference of a cylindrical lead. In other examples, one or more of leads 108 are linear leads having 8 ring electrodes along the axial length of the lead. In another example, the electrodes are segmented rings arranged in a linear fashion along the axial length of the lead and at the periphery of the lead.

The stimulation parameter set of a therapy stimulation program that defines the stimulation pulses of electrical stimulation therapy by IMD 110 through the electrodes of leads 108 may include information identifying which electrodes have been selected for delivery of stimulation according to a stimulation program, the polarities of the selected electrodes, i.e., the electrode combination for the program, voltage or current amplitude, pulse frequency, pulse width, pulse shape of stimulation delivered by the electrodes. These stimulation parameters values that make up the stimulation parameter set that defines pulses may be predetermined parameter values defined by a user and/or automatically determined by system 100 based on one or more factors or user input.

Although FIG. 1 is directed to SCS therapy, e.g., used to treat pain, in other examples system 100 may be configured to treat any other condition that may benefit from electrical stimulation therapy. For example, system 100 may be used to treat tremor, Parkinson's disease, epilepsy, a pelvic floor disorder (e.g., urinary incontinence or other bladder dysfunction, fecal incontinence, pelvic pain, bowel dysfunction, or sexual dysfunction), obesity, gastroparesis, or psychiatric disorders (e.g., depression, mania, obsessive compulsive disorder, anxiety disorders, and the like). In this manner, system 100 may be configured to provide therapy taking the form of deep brain stimulation (DBS), peripheral nerve stimulation (PNS), peripheral nerve field stimulation (PNFS), cortical stimulation (CS), pelvic floor stimulation, gastrointestinal stimulation, or any other stimulation therapy capable of treating a condition of patient 102.

In some examples, lead 108 includes one or more sensors configured to allow IMD 110 to monitor one or more parameters of patient 102, such as patient activity, pressure, temperature, or other characteristics. The one or more sensors may be provided in addition to, or in place of, therapy delivery by lead 108.

IMD 110 is generally configured to deliver electrical stimulation therapy to patient 102 via selected combinations of electrodes carried by one or both of leads 108, alone or in combination with an electrode carried by or defined by an outer housing of IMD 110. The target tissue for the electrical stimulation therapy may be any tissue affected by electrical stimulation, which may be in the form of electrical stimulation pulses or continuous waveforms. In some examples, the target tissue includes nerves, smooth muscle, or skeletal muscle. In the example illustrated by FIG. 1, the target tissue is tissue proximate spinal cord 106, such as within an intrathecal space or epidural space of spinal cord 106, or, in some examples, adjacent nerves that branch off spinal cord 106. Leads 108 may be introduced into spinal cord 106 in via any suitable region, such as the thoracic, cervical, or lumbar regions. Stimulation of spinal cord 106 may, for example, prevent pain signals from traveling through spinal cord 106 and to the brain of patient 102. Patient 102 may perceive the interruption of pain signals as a reduction in pain and, therefore, efficacious therapy results. In other examples, stimulation of spinal cord 106 may produce paresthesia which may be reduce the perception of pain by patient 102, and thus, provide efficacious therapy results.

IMD 110 is configured to generate and deliver electrical stimulation therapy to a target stimulation site within patient 102 via the electrodes of leads 108 to patient 102 according to one or more therapy stimulation programs. A therapy stimulation program defines values for one or more parameters (e.g., a parameter set) that define an aspect of the therapy delivered by IMD 110 according to that program. For example, a therapy stimulation program that controls delivery of stimulation by IMD 110 in the form of pulses may define values for voltage or current pulse amplitude, pulse width, pulse rate (e.g., pulse frequency), electrode combination, pulse shape, etc. for stimulation pulses delivered by IMD 110 according to that program.

A user, such as a clinician or patient 102, may interact with a user interface of an external programmer 104 to program IMD 110. Programming of IMD 110 may refer generally to the generation and transfer of commands, programs, or other information to control the operation of IMD 110. In this manner, IMD 110 may receive the transferred commands and programs from external programmer 104 to control stimulation, such as stimulation pulses that provide electrical stimulation therapy. For example, external programmer 104 may transmit therapy stimulation programs, stimulation parameter adjustments, therapy stimulation program selections, posture states, user input, or other information to control the operation of IMD 110, e.g., by wireless telemetry or wired connection.

In some cases, external programmer 104 may be characterized as a physician or clinician programmer if it is primarily intended for use by a physician or clinician. In other cases, external programmer 104 may be characterized as a patient programmer if it is primarily intended for use by a patient. A patient programmer may be generally accessible to patient 102 and, in many cases, may be a portable device that may accompany patient 102 throughout the patient's daily routine. For example, a patient programmer may receive input from patient 102 when the patient wishes to terminate or change electrical stimulation therapy, or when a patient perceives stimulation being delivered. In general, a physician or clinician programmer may support selection and generation of programs by a clinician for use by IMD 110, whereas a patient programmer may support adjustment and selection of such programs by a patient during ordinary use. In other examples, external programmer 104 may include, or be part of, an external charging device that recharges a power source of IMD 110. In this manner, a user may program and charge IMD 110 using one device, or multiple devices.

As described herein, information may be transmitted between external programmer 104 and IMD 110. Therefore, IMD 110 and external programmer 104 may communicate via wireless communication using any techniques known in the art. Examples of communication techniques may include, for example, radiofrequency (RF) telemetry and inductive coupling, but other techniques are also contemplated. In some examples, external programmer 104 includes a communication head that may be placed proximate to the patient's body near the IMD 110 implant site to improve the quality or security of communication between IMD 110 and external programmer 104. Communication between external programmer 104 and IMD 110 may occur during power transmission or separate from power transmission.

In some examples, IMD 110, in response to commands from external programmer 104, delivers electrical stimulation therapy according to a plurality of therapy stimulation programs to a target tissue site of the spinal cord 106 of patient 102 via electrodes (not depicted) on leads 108. In some examples, IMD 110 modifies therapy stimulation programs as therapy needs of patient 102 evolve over time. For example, the modification of the therapy stimulation programs may cause the adjustment of at least one parameter of the plurality of stimulation pulses. When patient 102 receives the same therapy for an extended period, the efficacy of the therapy may be reduced. In some cases, parameters of the plurality of stimulation pulses may be automatically updated.

As described herein, IMD 110 may be configured to detect ECAP signals which are representative of the number of nerve fibers activated by a delivered stimulation signal (e.g., a delivered pulse). However, IMD 110 may employ various techniques in order to appropriately detect the ECAP signal or one or more features of the ECAP signal that is elicited by the delivered stimulation pulse. The ECAP signal may include several features such as different peaks that include a first peak (P1), a trough (N1), a second peak (P2), and additional troughs and peaks in some situations. The detection of one or more features of the ECAP signal may be difficult as features of the ECAP signal may change in time with respect to the delivery of the stimulation pulse that elicits the ECAP signal. This change in the delay of the ECAP features from the delivered stimulation pulse can be referred to as latency. A change in latency of the features in the ECAP signal may be due to different nerve fibers being activated from differences in the parameter values of the stimulation pulse. For example, changing a parameter value for a stimulation pulse may change the ratio of slow nerve fibers to fast nerve fibers. Other factors may also cause the latency to be different for different parameter values.

IMD 110 may employ one or more sensing windows to identify respective features in each ECAP signal, but if a change in latency causes a feature to occur outside of the respective sensing window, IMD 110 may not identify the appropriate feature of the ECAP signal. A consequence of failing to identify the appropriate feature in the ECAP signal may result in an inability of IMD 110 to appropriate adjust electrical stimulation therapy based on the sensed ECAP signals. Therefore, IMD 110 may be configured to determine a sensing window for sensing one or more features of an ECAP signal based on a value of a stimulation parameter that at least partially defined the stimulation pulse that elicited the ECAP signal. For example, the latency of one or more (or all) features of ECAP signals may increase as the current amplitude increases for subsequent stimulation pulses. IMD 110 can then adjust, based on the value of the stimulation parameter that defines the stimulation pulse that elicited the ECAP signal, one or more aspects of a sensing window (e.g., the window duration or the start of the sensing window after the stimulation pulse) that IMD 110 uses to detect one or more features of the ECAP signal.

The relationship between stimulation pulse parameter values and latency of features in the ECAP signal may be patient-dependent. In other words, IMD 110 may select the sensing window to accommodate the specific way that the nerves of the patient react to different stimuli. Therefore, IMD 110 may determine the relationship between the latency and the stimulation parameter values of stimulation pulses delivered to the specific patient, as the relationship may be dependent on patient specific conditions such as electrode implant location, patient anatomy, disease state, neurological conditions, or any other patient-specific conditions that affect response to electrical stimulus. Prior to delivery of therapy, periodically over the course of therapy, or in response to a user or system request, the system may deliver a sweep of stimulation pulses with different parameter values and measure the latency between one or more features of the ECAP signal for each parameter value. IMD 110 can then generate the relationship between the parameter values and one or more aspects of the sensing window. IMD 110 can select appropriate sensing windows for subsequent sensing of ECAP signals based on the parameter values used to define the delivered stimulation pulse that elicits the ECAP signals. By selecting or adjusting one or more aspects of the sensing window in this manner, IMD 110 may be configured to detect the appropriate features from the ECAP signal that the system uses to generate the ECAP characteristic used for closed-loop control of stimulation therapy.

In one example, IMD 110 may be or be part of a system (e.g., system 100) that includes processing circuitry (e.g., processing circuitry 208 of FIG. 2) configured to perform functions for determining sensing windows for detecting one or more features of ECAP signals. IMD 110 may be configured to determine a value of a stimulation parameter that at least partially defines an electrical stimulation pulse and select, based on the value of the stimulation parameter, a sensing window for detecting one or more features of a sensed evoked compound action potential (ECAP) signal elicited by the electrical stimulation pulse. Using the sensing window, IMD 110 can determine a value of the one or more features within the sensing window from the sensed ECAP signal. IMD 110 may then control, based on the value of the one or more features, subsequent electrical stimulation deliverable to a patient.

IMD 110 may include a memory configured to store a relationship between the stimulation parameter and the sensing window. IMD 110 then be configured to select the sensing window based on the value of the stimulation parameter and the relationship stored in the memory. In some examples, the relationship indicates one or more stimulation parameter values defining the sensing window such that a first feature of the one or more features of the ECAP signal falls within the sensing window and a second feature of the ECAP signal does not fall within the sensing window. The relationship may be a linear or non-linear relationship between a stimulation parameter and an aspect of the sensing window. The relationship may be established for an aspect of the sensing window that is the duration, or temporal width, of the sensing window. In addition, or alternatively, the relationship may be established for an aspect of the sensing window that is the start time of the sensing window with respect to either the beginning of delivery of the stimulation pulse or the end of the delivery of the stimulation pulse. This start time for the sensing window may also be referred to as the delay or blanking period between the stimulation pulse and the beginning of the sensing window. In this manner, IMD 110 may select at least the duration of the sensing window or the start time of the sensing window to select one or more sensing windows for the ECAP signal.

In one example, the relationship may establish a linear transfer function that translates current amplitude of the stimulation pulse to a start time for the sensing window. In this manner, the sensing window defines a period of time within the ECAP signal during which IMD 110 can detect one or more features. The relationship may be established based at least in part on data collected from the patient. For example, IMD 110 may perform a calibration procedure during which IMD 110 performs a sweep of stimulation pulses defined by different values of a stimulation parameter, such as current amplitude. In other examples, IMD 110 may perform a sweep of stimulation pulses with a different stimulation parameter varied, such as pulse width, pulse shape, etc. The stimulation parameter varied may correspond to the stimulation parameter that will be varied as part of adjusting electrical stimulation during closed-loop stimulation.

The calibration procedure that IMD 110 may perform to determine the relationship between stimulation parameter values and parameters of the sensing window may include various steps. For example, IMD 110 may control the stimulation circuitry to deliver the plurality of electrical stimulation pulses as a sweep of pulses comprising iteratively increasing amplitude values of the stimulation parameter. IMD 110 can then determine a latency curve between the amplitude values of the stimulation parameter and a latency of the one or more features of respective ECAP signals elicited by respective electrical stimulation pulses of the plurality of electrical stimulation pulses. For example, the latency of a feature may be the period of time from delivery of the stimulation pulse to identification of the intended feature, such as the N1 trough in the ECAP signal. IMD 110 can then determine, based on the latency curve, a relationship between values of the stimulation parameter and one or more parameters of the sensing window. The relationship may then adjust for how the sensing window might be most appropriate to capture the feature according to the latency of that feature or other feature within the ECAP signal. Then, using the relationship, IMD 110 may control, based on the relationship and the first value of the stimulation parameter, the stimulation circuitry to deliver the subsequent electrical stimulation to the patient. IMD 110 may use the value(s) of the one or more features within the sensing window of the ECAP signal to generate an ECAP characteristic (e.g., an absolute value between two peaks or a peak and a trough) representative of the sensed ECAP signal.

Efficacy of electrical stimulation therapy (e.g., neural recruitment) may be indicated by one or more characteristics (e.g., an amplitude of or between one or more peaks or an area under the curve of one or more peaks) of an action potential that is evoked by a stimulation pulse delivered by IMD 110 (i.e., a characteristic value of the ECAP signal). Electrical stimulation therapy delivery by leads 108 of IMD 110 may cause neurons within the target tissue to evoke a compound action potential that travels up and down the target tissue, eventually arriving at sensing electrodes of IMD 110. Furthermore, stimulation may also elicit at least one ECAP signal, and ECAPs responsive to stimulation may also be a surrogate for the effectiveness of the therapy. The amount of action potentials (e.g., number of neurons propagating action potential signals) that are evoked may be based on the various parameters of electrical stimulation pulses such as amplitude, pulse width, frequency, pulse shape (e.g., slew rate at the beginning and/or end of the pulse), etc. In addition, the amount of action potentials that are evoked may vary depending on whether the intensity of stimulation pulses is increasing or decreasing from successive pulses. The slew rate may define the rate of change of the voltage and/or current amplitude of the pulse at the beginning and/or end of each pulse or each phase within the pulse. For example, a very high slew rate indicates a steep or even near vertical edge of the pulse, and a low slew rate indicates a longer ramp up (or ramp down) in the amplitude of the pulse. In some examples, these parameters contribute to an intensity of the electrical stimulation. In addition, a characteristic of the ECAP signal (e.g., an amplitude) may change based on the distance between the stimulation electrodes and the nerves subject to the electrical field produced by the delivered stimulation pulses.

Some example techniques for adjusting stimulation parameter values for stimulation pulses (e.g., pulses that may or may not contribute to therapy for the patient) are based on comparing the value of a characteristic of a measured ECAP signal to a target ECAP characteristic value. In response to delivering a stimulation pulse defined by a set of stimulation parameter values, IMD 110, via two or more electrodes interposed on leads 108, senses electrical potentials of tissue of the spinal cord 106 of patient 102 to measure the electrical activity of the tissue. IMD 110 senses ECAPs from the target tissue of patient 102, e.g., with electrodes on one or more leads 108 and associated sense circuitry. In some examples, IMD 110 receives a signal indicative of the ECAP from one or more sensors, e.g., one or more electrodes and circuitry, internal or external to patient 102. Such an example signal may include a signal indicating an ECAP of the tissue of patient 102. Examples of the one or more sensors include one or more sensors configured to measure a compound action potential of patient 102, or a physiological effect indicative of a compound action potential. For example, to measure a physiological effect of a compound action potential, the one or more sensors may be an accelerometer, a pressure sensor, a bending sensor, a sensor configured to detect a posture of patient 102, or a sensor configured to detect a respiratory function of patient 102. However, in other examples, external programmer 104 receives a signal indicating a compound action potential in the target tissue of patient 102 and transmits a notification to IMD 110.

In the example of FIG. 1, IMD 110 is described as performing a plurality of processing and computing functions. However, external programmer 104 instead may perform one, several, or all of these functions. In this alternative example, IMD 110 functions to relay sensed signals to external programmer 104 for analysis, and external programmer 104 transmits instructions to IMD 110 to adjust the one or more parameters defining the electrical stimulation therapy based on analysis of the sensed signals. For example, IMD 110 may relay the sensed signal indicative of an ECAP to external programmer 104. External programmer 104 may compare the parameter value of the ECAP to the target ECAP characteristic value, and in response to the comparison, external programmer 104 may instruct IMD 110 to adjust one or more stimulation parameter that defines the electrical stimulation informed pulses and, in some examples, control pulses, delivered to patient 102.

In some examples, the system changes the target ECAP characteristic value and/or growth rate(s) over a period of time, such as according to a change to a stimulation threshold (e.g., a perception threshold or detection threshold specific for the patient). The system may be programmed to change the target ECAP characteristic in order to adjust the intensity of informed pulses to provide varying sensations to the patient (e.g., increase or decrease the volume of neural activation). Although the system may change the target ECAP characteristic value, received ECAP signals may still be used by the system to adjust one or more parameter values of the informed pulses and/or control pulses in order to meet the target ECAP characteristic value.

One or more devices within system 100, such as IMD 110 and/or external programmer 104, may perform various functions as described herein. For example, IMD 110 may include stimulation circuitry configured to deliver electrical stimulation, sensing circuitry configured to sense a plurality ECAP signals, and processing circuitry. The processing circuitry may be configured to control the stimulation circuitry to deliver a plurality of electrical stimulation pulses having different amplitude values and control the sensing circuitry to detect, after delivery of each electrical stimulation pulse of the plurality of electrical stimulation pulses, a respective ECAP signal of the plurality of ECAP signals.

IMD 110 may modulate or adjust one or more stimulation parameters that at least partially define electrical stimulation, and IMD 110 may adjust the one or more stimulation parameters based on whether or not stimulation intensity is to be increased or decreased, and in some examples, also based on a detected posture state of the patient 102. For example, IMD 110 may select a gain value according to whether or not IMD 110 needs to increase or decrease stimulation intensity according to the detected ECAP characteristic value (e.g., an ECAP differential value indicating a positive or negative relationship between the detected ECAP characteristic value and a target ECAP characteristic value). IMD 110 may also use the detected posture state to determine how to employ ECAP signals in a closed-loop feedback system for adjusting stimulation parameters. In one example, IMD 110 includes stimulation generation circuitry configured to generate and deliver electrical stimulation to patient 102 according to one or more sets of stimulation parameters that at least partially define the pulses of the electrical stimulation. Each set of stimulation parameters may include at least one of an amplitude, a pulse width, a pulse frequency, or a pulse shape.

IMD 110 may include sensing circuitry configured to sense an ECAP signal elicited by delivered electrical stimulation, such as a stimulation pulse. IMD 110 may also include processing circuitry configured to control stimulation circuitry to deliver a first electrical stimulation pulse to patient 102 according to a first value of a stimulation parameter and determine a characteristic value of the ECAP signal elicited from the electrical stimulation. IMD 110 may then determine an ECAP differential value that indicates whether the characteristic value of the ECAP signal elicited by the first electrical stimulation pulse is one of greater than or equal to a selected ECAP characteristic value or less than the selected ECAP characteristic value. For example, IMD 110 may compare the characteristic value of the ECAP signal to the selected ECAP characteristic value, and the comparison may indicate whether IMD 110 may need to increase or decrease stimulation intensity of stimulation pulses in order to achieve the selected ECAP characteristic value (e.g., a target ECAP characteristic value).

Based on the ECAP differential value, IMD 110 may determine a gain value to use for adjusting a stimulation parameter value for subsequent stimulation pulses. For example, IMD 110 may select one gain value for adjusting a stimulation parameter value to increase stimulation intensity or select a different gain value for adjusting the stimulation parameter value to decrease stimulation intensity. Using the gain value, IMD 110 may then determine the parameter value that will at least partially define a second electrical stimulation pulse and control the stimulation circuitry to deliver the second electrical stimulation pulse according to the parameter value. In this manner, IMD 110 can use the selected gain value to determine one or more stimulation parameter values for the next stimulation pulses to be delivered to patient 102. In some examples, IMD 110 may adjust a previous stimulation parameter value to achieve the new parameter value based on the selected gain value.

When IMD 110 is configured to modulate stimulation pulses in order to maintain consistent nerve activation, such as increasing and decreasing a stimulation parameter to maintain a target ECAP characteristic value, IMD 110 may perform an example process. For example, IMD 110 may monitor an amplitude that is the characteristic value of the detected ECAP signal. IMD 110 may adjust the first value to the second value of the stimulation parameter by subtracting the amplitude from a target ECAP amplitude value for the patient to generate a differential amplitude (e.g., an ECAP differential value). The differential amplitude is the difference between the detected amplitude and the target ECAP amplitude value. IMD 110 may then multiply the differential amplitude by the gain value that at least partially defines the electrical stimulation to generate a differential value. The gain value may be a multiplier or fraction selected based on whether the ECAP differential value is positive or negative, and in some examples, the detected posture state. A larger gain value may be associated with posture states at which the distance between electrodes and the target nerve is larger because the distance causes less sensitivity for changes in stimulation pulse intensity. IMD 110 may then add the differential value to a previous amplitude value (e.g., the amplitude value of the last stimulation pulse that was delivered or elicited the ECAP signal) to generate the second value that at least partially defines the next stimulation pulses to be delivered to patient 102.

In other examples, IMD 110 may not attempt to maintain consistent nerve activation by modulating stimulation pulses to achieve a target ECAP characteristic value. Instead, IMD 110 may monitor characteristic values of ECAP signals any only take action when the characteristic value exceeds a threshold ECAP characteristic value. Characteristic values exceeding the threshold ECAP characteristic values may be indicative of increased stimulation perception that may be above an uncomfortable threshold or pain threshold for the patient. Therefore, reducing stimulation pulse intensity when the characteristic value exceeds this level of stimulation may reduce the likelihood that patient 102 experiences any uncomfortable sensations that may occur as a result of posture state changes or any transient movement. For example, IMD 110 may be configured to compare the characteristic value of the ECAP signal to a threshold ECAP characteristic value and determine that the characteristic value of the ECAP signal is greater than the threshold ECAP characteristic value (e.g., a positive ECAP differential value). Responsive to determining that the characteristic value of the ECAP signal is greater than the threshold ECAP characteristic value, IMD 110 may be configured to decrease the first value to the second value for the stimulation parameter of a subsequent stimulation pulse. As discussed above, IMD 110 may apply a gain value that is associated with the positive ECAP differential value or a negative ECAP differential value.

IMD 110 may continue to decrease the stimulation parameter value as long as the ECAP characteristic value continues to exceed the threshold ECAP characteristic value. Once, the stimulation parameter has been decreased, IMD 110 may attempt to increase the stimulation parameter value again back up to the predetermined first value intended for the stimulation pulses. IMD 110 may be configured to determine a other characteristic values of subsequent ECAP signals elicited from electrical stimulation pulses delivered after sensing the first ECAP signal. In response to determining that another characteristic value of the subsequent ECAP signals decreases below the threshold ECAP characteristic value, IMD 110 may then increase the value of the stimulation parameter back up to a value limited to be less than or equal to the first value (e.g., back up to the predetermined value for stimulation pulses that may be determined by a set of stimulation parameters or therapy program). Again, IMD 110 may use a different gain value to increase the stimulation parameter than the gain value used to decrease the stimulation parameter. In some examples, IMD 110 may iteratively increase the stimulation parameter value until the first value, or original value, is again reached after the characteristic values of the ECAP signal remain below the threshold ECAP characteristic value. IMD 110 may increase the stimulation parameter values at a slower rate than the stimulation parameter values are decreased, but, in other examples, IMD 110 may increase and decrease the stimulation parameters at the same rates.

As discussed herein, some example techniques for adjusting stimulation parameter values for electrical stimulation signals are based on comparing the value of a characteristic of a measured ECAP signal to a target ECAP characteristic value or using stimulation parameter values at a determined target ECAP characteristic to inform adjustment of one or more parameter values to maintain the target ECAP according to known relationships between parameters. For example, during delivery of an electrical stimulation signal, IMD 110, via two or more electrodes interposed on leads 108, senses electrical potentials of tissue of the spinal cord 106 of patient 102 to measure the electrical activity of the tissue. IMD 110 senses ECAPs from the target tissue of patient 102, e.g., with electrodes on one or more leads 108 and associated sensing circuitry. In some examples, IMD 110 receives a signal indicative of the ECAP from one or more sensors, e.g., one or more electrodes and circuitry, internal or external to patient 102. Such an example signal may include a signal indicating an ECAP of the tissue of the patient 102. Examples of the one or more sensors include one or more sensors can measure a compound action potential of the patient 102, or a physiological effect indicative of a compound action potential. For example, to measure a physiological effect of a compound action potential, the one or more sensors may be an accelerometer, a pressure sensor, a bending sensor, a sensor can detect a posture of patient 102, or a sensor can detect a respiratory function of patient 102. However, in other examples, external programmer 104 receives a signal indicating a compound action potential in the target tissue of patient 102 and transmits a notification to IMD 110.

In the example of FIG. 1, IMD 110 described as performing a plurality of processing and computing functions. However, external programmer 104 instead may perform one, several, or all of these functions. In this alternative example, IMD 110 functions to relay sensed signals to external programmer 104 for analysis, and external programmer 104 transmits instructions to IMD 110 to adjust the one or more parameters defining the electrical stimulation signal based on analysis of the sensed signals. For example, IMD 110 may relay the sensed signal indicative of an ECAP to external programmer 104. External programmer 104 may compare the parameter value of the ECAP to the target ECAP characteristic value, and in response to the comparison, external programmer 104 may instruct IMD 110 to adjust one or more parameters that define the electrical stimulation signal.

In the example techniques described herein, the stimulation parameter values, latency curves, and the target ECAP characteristic values (e.g., values of the ECAP indicative of target stimulation intensity) may be initially set at the clinic but may be set and/or adjusted at home by patient 102. Once the target ECAP characteristic values are set, the example techniques allow for automatic adjustment of stimulation parameters to maintain consistent volume of neural activation and consistent perception of therapy for the patient when the electrode-to-neuron distance changes. The ability to change the stimulation parameter values may also allow the therapy to have long term efficacy, with the ability to keep the intensity of the stimulation (e.g., as indicated by the ECAP) consistent by comparing the measured ECAP values to the target ECAP characteristic value. IMD 110 may perform these changes without intervention by a physician or patient 102.

In some examples, the system may change the target ECAP characteristic value over a period of time (e.g., based on a sensed posture state or change in patient conditions). The system may be programmed to change the target ECAP characteristic in order to adjust the intensity of the electrical stimulation signal to provide varying sensations to the patient (e.g., increase or decrease the volume of neural activation). In one example, a system may be programmed to oscillate a target ECAP characteristic value between a maximum target ECAP characteristic value and a minimum target ECAP characteristic value at a predetermined frequency to provide a sensation to the patient that may be perceived as a wave or other sensation that may provide therapeutic relief for the patient. The maximum target ECAP characteristic value, the minimum target ECAP characteristic value, and the predetermined frequency may be stored in the memory of IMD 110 and may be updated in response to a signal from external programmer 104 (e.g., a user request to change the values stored in the memory of IMD 110). In other examples, the target ECAP characteristic value may be programed to steadily increase or steadily decrease to a baseline target ECAP characteristic value over a period of time. In other examples, external programmer 104 may program the target ECAP characteristic value to automatically change over time according to other predetermined functions or patterns. In other words, the target ECAP characteristic value may be programmed to change incrementally by a predetermined amount or predetermined percentage, the predetermined amount or percentage being selected according to a predetermined function (e.g., sinusoid function, ramp function, exponential function, logarithmic function, or the like). Increments in which the target ECAP characteristic value is changed may be changed for every certain number of pulses or a certain unit of time. Although the system may change the target ECAP characteristic value, received ECAP signals may still be used by the system to adjust one or more parameter values of the electrical stimulation signal in order to meet the target ECAP characteristic value.

In some examples, IMD 110 may not be able to measure ECAPs from stimulation that has certain pulse widths and/or pulse frequencies. For example, longer pulse widths and higher pulse frequencies may result in a delivered stimulation pulse overlapping with an ECAP. Since the ECAP amplitude can be much lower amplitude than the stimulation pulse, the stimulation pulse can cover up any ECAP characteristic value of the signal. However, IMD 110 may use measured ECAPs at short pulse widths and/or lower pulse frequencies to identify a combination of stimulation parameter values that produce an ECAP characteristic value (e.g., intensity) that is representative of effective therapy. IMD 110 may then select longer pulse widths and/or higher pulse frequencies according to the relationship between the pulse width and pulse frequency that are estimated to produce a similar ECAP characteristic value that resulted in the effective therapy. In this manner, IMD 110 may measure ECAP signals elicited by some stimulation pulses (e.g., control pulses) and use the ECAP characteristic value derived thereof to inform adjustments to subsequent stimulation pulses (e.g., informed pulses) that have different stimulation parameters. The informed pulses may be configured to produce a therapeutic effect. The control pulses may, or may not, produce a therapeutic effect. In some examples, the control pulses may have the same or longer pulse width than the informed pulses.

Although in one example IMD 110 takes the form of an SCS device, in other examples, IMD 110 takes the form of any combination of deep brain stimulation (DBS) devices, implantable cardioverter defibrillators (ICDs), pacemakers, cardiac resynchronization therapy devices (CRT-Ds), left ventricular assist devices (LVADs), implantable sensors, orthopedic devices, or drug pumps, as examples.

FIG. 2 is a block diagram of IMD 200. IMD 200 may be an example of IMD 110 of FIG. 1. In the example shown in FIG. 2, IMD 200 includes stimulation generation circuitry 204, sensing circuitry 206, processing circuitry 208, sensor 210, telemetry circuitry 212, power source 214, and memory 216. Each of these circuits may be or include programmable or fixed function circuitry can perform the functions attributed to respective circuitry. For example, processing circuitry 208 may include fixed-function or programmable circuitry, stimulation generation circuitry 204 may include circuitry can generate electrical stimulation signals such as pulses or continuous waveforms on one or more channels, sensing circuitry 206 may include sensing circuitry for sensing signals, and telemetry circuitry 212 may include telemetry circuitry for transmission and reception of signals. Memory 216 may store computer-readable instructions that, when executed by processing circuitry 208, cause IMD 200 to perform various functions described herein. Memory 216 may be a storage device or other non-transitory medium.

In the example shown in FIG. 2, memory 216 stores patient posture state data 218, which may include one or more patient postures, an activity level, or a combination of patient posture and activity level. A set of pre-established posture state definitions for a patient may be stored in patient posture state data 218. A posture state definition may be modified based on user therapy adjustments and/or posture state information. In some cases, the posture state may be expanded and split, or instead, may be reduced in size based on posture state information. The posture state definitions can be automatically updated or updated by a patient, including creating new posture states. Posture states may include, for example, a supine posture, a prone posture, a lying left and/or lying right, a sitting posture, a reclining posture, a standing posture, and/or activities such as running or riding in an automobile.

Memory 216 may store stimulation parameter settings 220 within memory 216 or separate areas within memory 216. Each stored stimulation parameter setting 220 defines values for a set of electrical stimulation parameters (e.g., a stimulation parameter set or therapy program), such as pulse amplitude, pulse width, pulse frequency, electrode combination, pulse burst rate, pulse burst duration, and/or waveform shape. Stimulation parameter settings 220 may also include additional information such as instructions regarding delivery of electrical stimulation signals based on stimulation parameter relationship data, which can include relationships between two or more stimulation parameters based upon data from electrical stimulation signals delivered to patient 102 or data transmitted from external programmer 104. The stimulation parameter relationship data may include measurable aspects associated with stimulation, such as an ECAP characteristic value. Stimulation parameter settings 220 may also include target ECAP characteristics and/or threshold ECAP characteristic values determined for the patient and/or a history of measured ECAP characteristic values for the patient.

Memory 216 also stores patient calibration instructions characteristics 222 which may include instructions on calibrating growth curves, such as defining stimulation pulse sweeps in order to generate the relationships between ECAP characteristic values and one or more stimulation parameters, or latency curves, such as defining stimulation pulse sweeps in order to generate the relationships between ECAP signal feature latency and stimulation parameter values. Memory 216 may also store latency data 224 in separate areas from or as part of patient stimulation parameter settings. Latency data 224 may include raw data of the relationships between feature latencies and stimulation parameter values, relationships between stimulation parameter values and one or more aspects of sensing windows, or other data used to determine or select sensing windows for sensing one or more features of ECAP signals. In other examples, latency data 224 may include information regarding relationships between stimulation parameter values and sensing window aspects for one or more posture states. For example, the latency may be dependent on posture of the patient in addition to stimulation parameter values.

Accordingly, in some examples, stimulation generation circuitry 204 generates electrical stimulation signals (e.g., pulses) in accordance with the electrical stimulation parameters noted above. Other ranges of stimulation parameter values may also be useful and may depend on the target stimulation site within patient 102. While stimulation pulses are described, stimulation signals may be of any form, such as continuous-time signals (e.g., sine waves) or the like. Stimulation generation circuitry 204 may include independently controllable current sinks and sources for respective electrodes 232, 234. For example, stimulation generation circuitry 204 comprises a plurality of pairs of voltage sources, current sources, voltage sinks, or current sinks connected to each of electrodes 232, 234 such that each pair of electrodes has a unique signal circuit. In other words, in these examples, each of electrodes 232, 234 is independently controlled via its own signal circuit (e.g., via a combination of a regulated voltage source and sink or regulated current source and sink), as opposed to switching signals between electrodes 232, 234. In this manner, processing circuitry 208 may control switches or transistors to selective couple the sources and/or sinks to the conductor of electrodes of an electrode combination.

One or more switches (not shown) may selectively couple sensing circuitry 206 to respective electrodes in order to sense signals via two or more electrodes 232, 234. In other examples, switch circuitry may include one or more switch arrays, one or more multiplexers, one or more switches (e.g., a switch matrix or other collection of switches), or other electrical circuitry configured to direct stimulation signals from stimulation generation circuitry 204 to one or more of electrodes 232, 234, or directed sensed signals from one or more of electrodes 232, 234 to sensing circuitry 206. In other examples, stimulation generation circuitry 204 and/or sensing circuitry 206 may include sensing circuitry to direct signals to and/or from one or more of electrodes 232, 234, which may or may not also include switch circuitry.

Sensing circuitry 206 may be configured to monitor signals from any combination of electrodes 232, 234. In some examples, sensing circuitry 206 includes one or more amplifiers, filters, and analog-to-digital converters. Sensing circuitry 206 may be used to sense physiological signals, such as ECAPs. In some examples, sensing circuitry 206 detects ECAPs from a particular combination of electrodes 232, 234. In some cases, the particular combination of electrodes for sensing ECAPs includes different electrodes than a set of electrodes 232, 234 used to deliver stimulation pulses. Alternatively, in other cases, the particular combination of electrodes used for sensing ECAPs includes at least one of the same electrodes as a set of electrodes used to deliver stimulation pulses to patient 102. Sensing circuitry 206 may provide signals to an analog-to-digital converter, for conversion into a digital signal for processing, analysis, storage, or output by processing circuitry 208.

Processing circuitry 208 may include any one or more of a microprocessor, a controller, a digital signal processor (DSP), an application specific integrated circuit (ASIC), a field-programmable gate array (FPGA), discrete logic circuitry, or any other processing circuitry can provide the functions attributed to processing circuitry 208 herein may be embodied as firmware, hardware, software, or any combination thereof. Processing circuitry 208 controls stimulation generation circuitry 204 to generate electrical stimulation signals according to stimulation parameter settings 220 stored in memory 216 to apply stimulation parameter values, such as pulse amplitude, pulse width, pulse frequency, and waveform shape of each of the electrical stimulation signals.

In the example shown in FIG. 2, the set of electrodes 232 includes electrodes 232A, 232B, 232C, and 232D, and the set of electrodes 234 includes electrodes 234A, 234B, 234C, and 234D. In other examples, a single lead may include all eight electrodes 232 and 234 along a single axial length of the lead. Processing circuitry 208 also controls stimulation generation circuitry 204 to generate and apply the electrical stimulation signals to selected combinations of electrodes 232, 234. In other examples, stimulation generation circuitry 204 includes a switch circuit that may couple stimulation signals to selected conductors within leads 230, which, in turn, deliver the stimulation signals across selected electrodes 232, 234. Such a switch circuit may be a switch array, switch matrix, multiplexer, or any other type of switch circuitry can selectively couple stimulation energy to selected electrodes 232, 234 and to selectively sense bioelectrical neural signals of a spinal cord of the patient (not shown in FIG. 2) with selected electrodes 232, 234.

Electrodes 232, 234 on respective leads 230 may be constructed of a variety of different designs. For example, one or both of leads 230 may include one or more electrodes at each longitudinal location along the length of the lead, such as one electrode at different perimeter locations around the perimeter of the lead at each of the locations A, B, C, and D. In one example, the electrodes may be electrically coupled to stimulation generation circuitry 204, e.g., via switch circuitry 202 and/or switch circuitry of the stimulation generation circuitry 204, via respective wires that are straight or coiled within the housing of the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 230. These and other constructions may be used to create a lead with a complex electrode geometry.

Although sensing circuitry 206 is incorporated into a common housing with stimulation generation circuitry 204 and processing circuitry 208 in FIG. 2, in other examples, sensing circuitry 206 may be in a separate housing from IMD 200 and may communicate with processing circuitry 208 via wired or wireless communication techniques. In some examples, one or more of electrodes 232 and 234 may be suitable for sensing ECAPs. For instance, electrodes 232 and 234 may sense the voltage amplitude of a portion of the ECAP signals, where the sensed voltage amplitude is a characteristic the ECAP signal.

Memory 216 may be configured to store information within IMD 200 during operation. Memory 216 may include a computer-readable storage medium or computer-readable storage device. In some examples, memory 216 includes one or more of a short-term memory or a long-term memory. Memory 216 may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, memory 216 is used to store data indicative of instructions for execution by processing circuitry 208. As discussed herein, memory 216 can store patient posture state data 218, stimulation parameter settings 220, calibration instructions 222, and latency data 224.

Sensor 210 may include one or more sensing elements that sense values of a respective patient parameter. As described, electrodes 232 and 234 may be the electrodes that sense, via sensing circuitry 206, a value of the ECAP indicative of a target stimulation intensity at least partially caused by a set of stimulation parameter values. Sensor 210 may include one or more accelerometers, optical sensors, chemical sensors, temperature sensors, pressure sensors, or any other types of sensors. Sensor 210 may output patient parameter values that may be used as feedback to control delivery of electrical stimulation signals. IMD 200 may include additional sensors within the housing of IMD 200 and/or coupled via one of leads 108 or other leads. In addition, IMD 200 may receive sensor signals wirelessly from remote sensors via telemetry circuitry 212, for example. In some examples, one or more of these remote sensors may be external to patient (e.g., carried on the external surface of the skin, attached to clothing, or otherwise positioned external to the patient). In some examples, signals from sensor 210 may indicate a posture state (e.g., sleeping, awake, sitting, standing, or the like), and processing circuitry 208 may select target and/or threshold ECAP characteristic values according to the indicated posture state. In this manner, processing circuitry 208 may be configured to determine the currently occupied posture state of patient 102.

Telemetry circuitry 212 supports wireless communication between IMD 200 and an external programmer (not shown in FIG. 2) or another computing device under the control of processing circuitry 208. Processing circuitry 208 of IMD 200 may receive, as updates to programs, values for various stimulation parameters such as amplitude and electrode combination, from the external programmer via telemetry circuitry 212. Updates to stimulation parameter settings 220 and input efficacy threshold settings 226 may be stored within memory 216. Telemetry circuitry 212 in IMD 200, as well as telemetry circuits in other devices and systems described herein, such as the external programmer, may accomplish communication by radiofrequency (RF) communication techniques. In addition, telemetry circuitry 212 may communicate with an external medical device programmer (not shown in FIG. 2) via proximal inductive interaction of IMD 200 with the external programmer. The external programmer may be one example of external programmer 104 of FIG. 1. Accordingly, telemetry circuitry 212 may send information to the external programmer on a continuous basis, at periodic intervals, or upon request from IMD 110 or the external programmer.

Power source 214 delivers operating power to various components of IMD 200. Power source 214 may include a rechargeable or non-rechargeable battery and a power generation circuit to produce the operating power. Recharging may be accomplished through proximal inductive interaction between an external charger and an inductive charging coil within IMD 200. In other examples, traditional primary cell batteries may be used. In some examples, processing circuitry 208 may monitor the remaining charge (e.g., voltage) of power source 214 and select stimulation parameter values that may deliver similarly effective therapy at lower power consumption levels when needed to extend the operating time of power source 214. For example, power source 214 may switch to a lower pulse frequency based on the relationships of parameters that may provide similar ECAP characteristic values.

According to the techniques of the disclosure, stimulation generation circuitry 204 of IMD 200 receives, via telemetry circuitry 212, instructions to deliver electrical stimulation according to stimulation parameter settings 220 to a target tissue site of the spinal cord of the patient via a plurality of electrode combinations of electrodes 232, 234 of leads 230 and/or a housing of IMD 200. Each electrical stimulation signal may elicit an ECAP that is sensed by sensing circuitry 206 via electrodes 232 and 234. Processing circuitry 208 may receive, via an electrical signal sensed by sensing circuitry 206, information indicative of an ECAP signal (e.g., a numerical value indicating a characteristic of the ECAP in electrical units such as voltage or power) produced in response to the electrical stimulation signal(s). Stimulation parameter settings 220 may be updated according to the ECAPs recorded at sensing circuitry 206 according to the following techniques.

In one example, the plurality of pulses each have a pulse width of greater than approximately 300 µs and less than approximately 2000 µs (i.e., 2 milliseconds). In some examples, the pulse width is greater than approximately 300 µs and less than approximately 900 µs. In another example, the pulse width is greater than approximately 300 µs and less than approximately 500 µs. However, in other examples, pulses may have a pulse width less than 300 µs. In one example, the pulses have a pulse width of approximately 450 µs and a pulse frequency of approximately 60 Hertz. Amplitude (current and/or voltage) for the pulses may be between approximately 0.5 mA (or volts) and approximately 10 mA (or volts), although amplitude may be lower or greater in other examples.

In one example, the predetermined pulse frequency of the plurality of pulses may be less than approximately 400 Hertz. In some examples, the predetermined pulse frequency of the plurality of pulses may be between approximately 50 Hertz and 70 Hertz. In one example, the predetermined pulse frequency of the plurality of pulses may be approximately 60 Hertz. However, the pulses may have frequencies greater than 400 Hertz or less than 50 Hertz in other examples. In addition, the pulses may be delivered in bursts of pulses, with interburst frequencies of the pulses being low enough such that a sensed ECAP can still fit within the window between consecutive pulses delivered within the burst of pulses. In any example, processing circuity 208 may be configured to detect ECAPs elicited from respective stimulation pulses.

Processing circuitry 208 may be configured to compare one or more characteristics of ECAPs sensed by sensing circuitry 206 with target ECAP characteristics stored in memory 216 (e.g., patient ECAP characteristics 222). For example, processing circuitry 208 can determine the amplitude of each ECAP signal received at sensing circuitry 206, and processing circuitry 208 can determine the representative amplitude of at least one respective ECAP signal and compare the representative amplitude of a series of ECAP signals to a target ECAP.

In other examples, processing circuitry 208 may use the representative amplitude of the at least one respective ECAP to change other parameters of stimulation pulses to be delivered, such as pulse width, pulse frequency, and pulse shape. All of these parameters may contribute to the intensity of the stimulation pulses, and changing one or more of these parameter values may effectively adjust the stimulation pulse intensity to compensate for the changed distance between the stimulation electrodes and the nerves indicated by the characteristic (e.g., a representative amplitude) of the ECAP signals.

In some examples, leads 230 may be linear 8-electrode leads (not pictured); sensing and stimulation delivery may each be performed using a different set of electrodes. In a linear 8-electrode lead, each electrode may be numbered consecutively from 0 through 7. For instance, a pulse may be generated using electrode 1 as a cathode and electrodes 0 and 2 as anodes (e.g., a guarded cathode), and a respective ECAP signal may be sensed using electrodes 6 and 7, which are located on the opposite end of the electrode array. This strategy may minimize the interference of the stimulation pulse with the sensing of the respective ECAP. Other electrode combinations may be implemented, and the electrode combinations may be changed using the patient programmer via telemetry circuitry 212. For example, stimulation electrodes and sensing electrodes may be positioned closer together. Shorter pulse widths for the nontherapeutic pulses may allow the sensing electrodes to be closer to the stimulation electrodes.

In one example, sensor 210 may detect a change in posture state, including activity or a change in posture of the patient. Processing circuitry 208 may receive an indication from sensor 210 that the activity level or posture of the patient is changed, and processing circuitry 208 can initiate or change the delivery of the plurality of pulses according to stimulation parameter settings 220. For example, processing circuitry 208 may increase the frequency of pulse delivery and respective ECAP sensing in response to receiving an indication that the patient activity has increased, which may indicate that the distance between electrodes and nerves will likely change. Alternatively, processing circuitry 208 may decrease the frequency of pulse delivery and respective ECAP sensing in response to receiving an indication that the patient activity has decreased. In some examples, one or more therapy parameters (e.g., frequency, amplitude, slew rate, pulse width, or the like) may be adjusted (e.g., increased or decreased) in response to receiving an indication that the patient posture state has changed. Processing circuitry 208 can update patient posture state data 218 and latency data 224 according to the signal received from sensor 210.

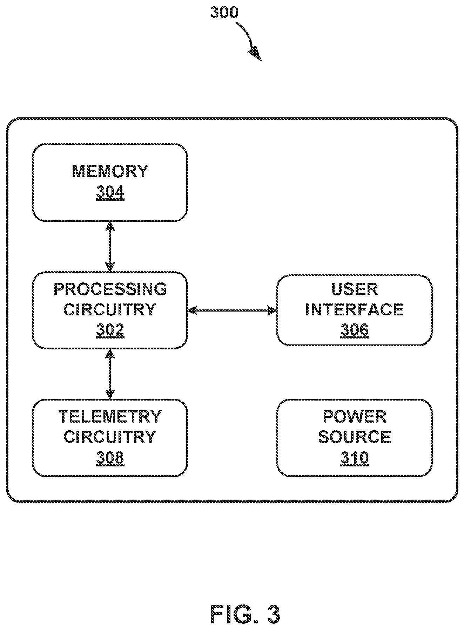

FIG. 3 is a block diagram of the example external programmer 300. External programmer 300 may be an example of external programmer 104 of FIG. 1. Although programmer 300 may generally be described as a hand-held device, external programmer 300 may be a larger portable device or a more stationary device. In addition, in some examples, external programmer 300 may be included as part of an external charging device or include the functionality of an external charging device. As illustrated in FIG. 3, external programmer 300 may include a processing circuitry 302, memory 304, user interface 306, telemetry circuitry 308, and power source 310. Storage device 304 may store instructions that, when executed by processing circuitry 302, cause processing circuitry 302 and external programmer 300 to provide the functionality ascribed to external programmer 300 throughout this disclosure. Each of these components, circuitry, or modules, may include electrical circuitry that can perform some, or all of the functionality described herein. For example, processing circuitry 302 may include processing circuitry to perform the processes discussed with respect to processing circuitry 302.

In general, programmer 300 comprises any suitable arrangement of hardware, alone or in combination with software and/or firmware, to perform the techniques attributed to programmer 300, and processing circuitry 302, user interface 306, and telemetry circuitry 308 of programmer 300. In various examples, programmer 300 may include one or more processors, such as one or more microprocessors, DSPs, ASICs, FPGAs, or any other equivalent integrated or discrete logic circuitry, as well as any combinations of such components. Programmer 300 also, in various examples, may include a memory 304, such as RAM, ROM, PROM, EPROM, EEPROM, flash memory, a hard disk, a CD-ROM, comprising executable instructions for causing the one or more processors to perform the actions attributed to them. Moreover, although processing circuitry 302 and telemetry circuitry 308 are described as separate, in some examples, processing circuitry 302 and telemetry circuitry 308 are functionally integrated. In some examples, processing circuitry 302 and telemetry circuitry 308 correspond to individual hardware units, such as ASICs, DSPs, FPGAs, or other hardware units.

Memory 304 (e.g., a storage device) may store instructions that, when executed by processing circuitry 302, cause processing circuitry 302 and programmer 300 to provide the functionality ascribed to programmer 300 throughout this disclosure. For example, memory 304 may include instructions that cause processing circuitry 302 to obtain a stimulation parameter setting from memory, select a spatial electrode movement pattern, or receive a user input and send a corresponding command to programmer 300, or instructions for any other functionality. In addition, memory 304 may include a plurality of stimulation parameter settings, where each setting includes a parameter set that defines electrical stimulation. Memory 304 may also store data received from a medical device (e.g., IMD 110). For example, memory 304 may store ECAP related data recorded at a sensing circuitry of the medical device, and memory 304 may also store data from one or more sensors of the medical device.

User interface 306 may include a button or keypad, lights, a speaker for voice commands, a display, such as a liquid crystal (LCD), light-emitting diode (LED), or organic light-emitting diode (OLED). In some examples the display may be a touch screen. User interface 306 can display any information related to the delivery of electrical stimulation, identified patient behaviors, sensed patient parameter values, patient behavior criteria, or any other such information. External programmer 300 may receive user input (e.g., indication of when the patient changes posture states) via user interface 306. The input may be, for example, in the form of pressing a button on a keypad or selecting an icon from a touch screen. The input may request starting or stopping electrical stimulation, the input may request a new spatial electrode movement pattern or a change to an existing spatial electrode movement pattern, of the input may request some other change to the delivery of electrical stimulation. In other examples, user interface 306 may receive input from the patient and/or clinician regarding efficacy of the therapy, such as binary feedback, numerical ratings, textual input, etc. In some examples, processing circuitry 302 may interpret patient requests to change therapy as negative feedback regarding the current parameter values used to define therapy.

Telemetry circuitry 308 may support wireless communication between the medical device and programmer 300 under the control of processing circuitry 302. Telemetry circuitry 308 can communicate with another computing device via wireless communication techniques, or direct communication through a wired connection. In some examples, telemetry circuitry 308 provides wireless communication via an RF or proximal inductive medium. In some examples, telemetry circuitry 308 includes an antenna, which may take on a variety of forms, such as an internal or external antenna.

Examples of local wireless communication techniques that may be employed to facilitate communication between programmer 300 and IMD 110 include RF communication according to the 802.11 or Bluetooth specification sets or other standard or proprietary telemetry protocols. In this manner, other external devices may be capable of communicating with programmer 300 without needing to establish a secure wireless connection. As described herein, telemetry circuitry 308 can transmit a spatial electrode movement pattern or other stimulation parameter values to IMD 110 for delivery of electrical stimulation.

In some examples, selection of stimulation parameter settings may be transmitted to the medical device for delivery to the patient. In other examples, stimulation parameter settings may include medication, activities, or other instructions that the patient must perform themselves or a caregiver perform for patient 102. In some examples, external programmer 300 may provide visual, audible, and/or tactile notifications that indicate there are new instructions. External programmer 300 may require receiving user input acknowledging that the instructions have been completed in some examples.

According to the techniques of the disclosure, user interface 306 of external programmer 300 receives an indication from a clinician instructing a processor of the medical device to update one or more stored values, such as requesting recalibration of the relationships between stimulation parameter values and ECAP signal feature latencies, patient posture state settings, gain values, growth curve settings, or stimulation parameter settings. User interface 306 may also receive instructions from the clinician commanding any electrical stimulation.

Power source 310 can deliver operating power to various components of programmer 300. Power source 310 may be the same as or substantially similar to power source 214. Power source 310 may include a battery and a power generation circuit to produce the operating power. In some examples, the battery is rechargeable to allow extended operation. Recharging may be accomplished by electrically coupling power source 310 to a cradle or plug that is connected to an alternating current (AC) outlet. In addition, recharging may be accomplished through proximal inductive interaction between an external charger and an inductive charging coil within external programmer 300. In other examples, traditional batteries (e.g., nickel cadmium or lithium ion batteries) may be used. In addition, external programmer 300 may be directly coupled to an alternating current outlet to operate.

The architecture of external programmer 300 illustrated in FIG. 3 is shown as an example. The techniques as set forth in this disclosure may be implemented in the example external programmer 300 of FIG. 3, as well as other types of systems not described specifically herein. Nothing in this disclosure should be construed so as to limit the techniques of this disclosure to the example architecture illustrated by FIG. 3.

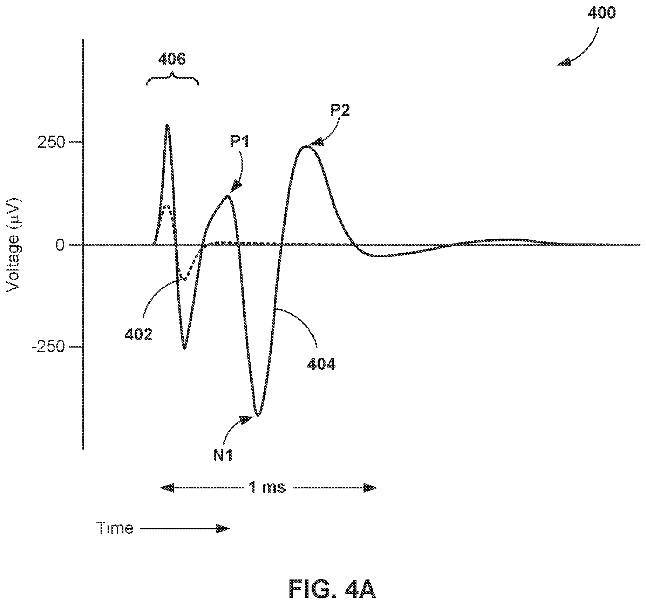

FIG. 4A is a graph 400 of an example ECAP signals sensed for respective electrical stimulation pulses. As shown in FIG. 4A, graph 400 shows example ECAP signal 402 (dotted line) and ECAP signal 404 (solid line). Each of ECAP signals 402 and 404 may be sensed from pulses that were delivered from a guarded cathode and bi-phasic pulses including an interphase interval between each positive and negative phase of the pulse. The guarded cathode of the stimulation electrodes is located at the end of an 8-electrode lead while two sensing electrodes are provided at the other end of the 8-electrode lead. ECAP signal 402 illustrates the voltage amplitude sensed as a result from a sub-threshold stimulation pulse. Peaks 406 of ECAP signal 402 are detected and represent the artifact of the delivered pulse. However, no propagating signal is detected after the artifact in ECAP signal 404 because the pulse was sub-threshold.

In contrast to ECAP signal 402, ECAP signal 404 represents the voltage amplitude detected from a supra-threshold stimulation pulse. Peaks 406 of ECAP signal 404 are detected and represent the artifact of the delivered pulse. After peaks 406, ECAP signal 404 also includes various features, which include peaks P1, N1, and P2, which are three peaks representative of propagating action potentials from an ECAP. In some examples, N1 may be referred to as a negative peak or trough instead. The example duration of the artifact and peaks P1, N1, and P2 is approximately 1 millisecond (ms). When detecting the ECAP of ECAP signal 404, different characteristics may be identified. For example, the characteristic of the ECAP may be the amplitude between features N1 and P2. This N1-P2 amplitude can be detected even if the artifact impinges on P1, a relatively large signal, and the N1-P2 amplitude may be minimally affected by electronic drift in the signal. In other examples, the characteristic of the ECAP used to control pulses may be an amplitude of P1, N1, or P2 with respect to neutral or zero voltage. In some examples, the characteristic of the ECAP used to control pulses may be a sum of two or more of peaks P1, N1, or P2. In other examples, the characteristic of ECAP signal 404 may be the area under one or more of peaks P1, N1, and/or P2. In other examples, the characteristic of the ECAP may be a ratio of one of peaks P1, N1, or P2 to another one of the peaks. In some examples, the characteristic of the ECAP may be a slope between two points in the ECAP signal, such as the slope between N1 and P2. In other examples, the characteristic of the ECAP may be the time between two points of the ECAP, such as the time between N1 and P2. The time between two points in the ECAP signal (e.g., the beginning of stimulation pulse of peaks 406 and N1) may be referred to as a latency of the ECAP and may indicate the types of fibers being captured by the pulse. ECAP signals with lower latency (i.e., smaller latency values) indicate a higher percentage of nerve fibers that have faster propagation of signals, whereas ECAP signals with higher latency (i.e., larger latency values) indicate a higher percentage of nerve fibers that have slower propagation of signals. Other characteristics of the ECAP signal may be used in other examples.

The amplitude of the ECAP signal increases with increased amplitude of the pulse, as long as the pulse amplitude is greater than the threshold such that nerves depolarize and propagate the signal. The target ECAP characteristic (e.g., the target ECAP amplitude) may be determined from the ECAP signal detected from a pulse when pulses are determined to deliver effective therapy to the patient. The ECAP signal thus is representative of the distance between the stimulation electrodes and the nerves appropriate for the stimulation parameter values of the pulses delivered at that time. Therefore, IMD 110 may attempt to use detected changes to the measured ECAP characteristic value to change stimulation pulse parameter values and maintain the target ECAP characteristic value during stimulation pulse delivery. Alternatively, IMD 110 may attempt to prevent undesirable stimulation intensity by decreasing stimulation pulse intensity in response to the ECAP characteristic value exceeding a threshold ECAP characteristic value.

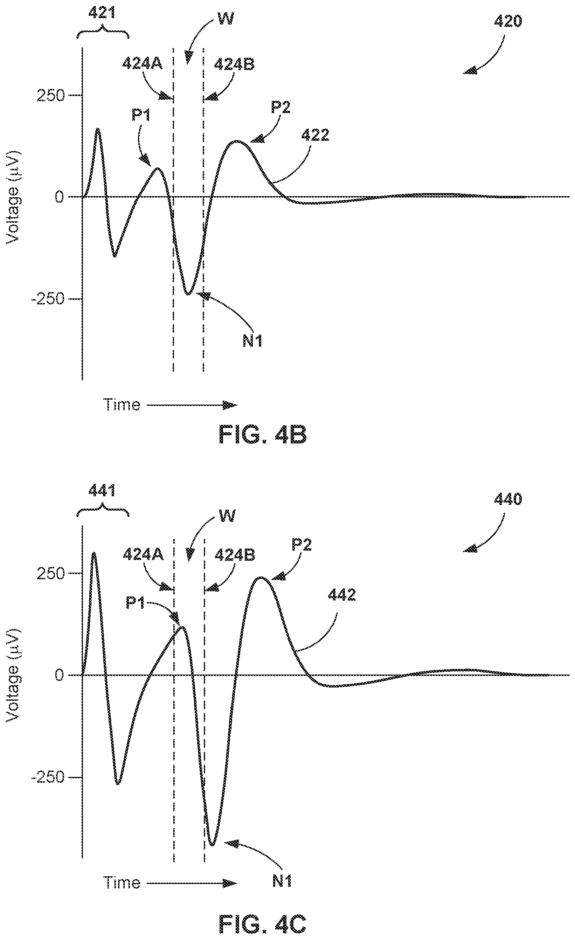

FIGS. 4B and 4C are graphs of example ECAP signals sensed from stimulation pulses having different current amplitudes Graph 420 of FIG. 4B is similar to graph 440 of FIG. 4C, but graph 440 indicates how the latency of an ECAP signal (e.g., ECAP signal 442) increases as a result of a higher amplitude stimulation pulse 441. As shown in FIG. 4B, ECAP signal 442 includes stimulation pulse 421 and the following features of the ECAP signal which include P1, N1, and P2. Sensing window W is shown as starting at time 424A after the start of stimulation pulse 421 and ending at time 424B. Therefore, the duration of sensing window W is the time period between times 424A and 424B. If the feature of ECAP signal 422 intended to be captured by sensing window W is N1, then the system will correctly identify N1. Therefore, sensing window W is correctly sized and positioned to identify the feature N1 in ECAP signal 422.

However, graph 440 of FIG. 4C illustrates what happens if a stimulation parameter of the stimulation pulse changes. Stimulation pulse 441 is shown to have a much larger current amplitude than stimulation pulse 421. This larger current amplitude of stimulation pulse 441 causes the latency to increase for features of ECAP signal 442 as compared to the smaller current amplitude of stimulation pulse 421. For example, sensing window W terminates too early for N1 to be identified because time 424B lies prior to the peak of N1. As described herein, the system may compensate for this increased latency for larger current amplitudes by shifting the sensing window later in time in order to correctly identify N1 within the sensing window. It is noted that for some patients, increased latency may correspond to smaller current amplitudes, or different changes to other stimulation parameters. Therefore, the relationships between sensing windows and stimulation parameter values may be determined for each patient.

In some examples, the duration of sensing window W may stay the same, but the sensing window may move closer or further away from the delivery of the stimulation pulse. The sensing window may have any duration appropriate to identify a target feature of the ECAP signal. In some examples, the sensing window may have a duration selected from 50 µs to 800 µs. Different windows may be used depending on the particular target feature being assessed. In one specific example, a window of 300 µs is used to identify the N1 peak and another window of 400 µs is used to identify the P2 peak. However, the window may be selected to have a duration shorter or longer than any of these examples. In any case, the sensing window duration may be adjusted based on the assessed ECAP latency as well.

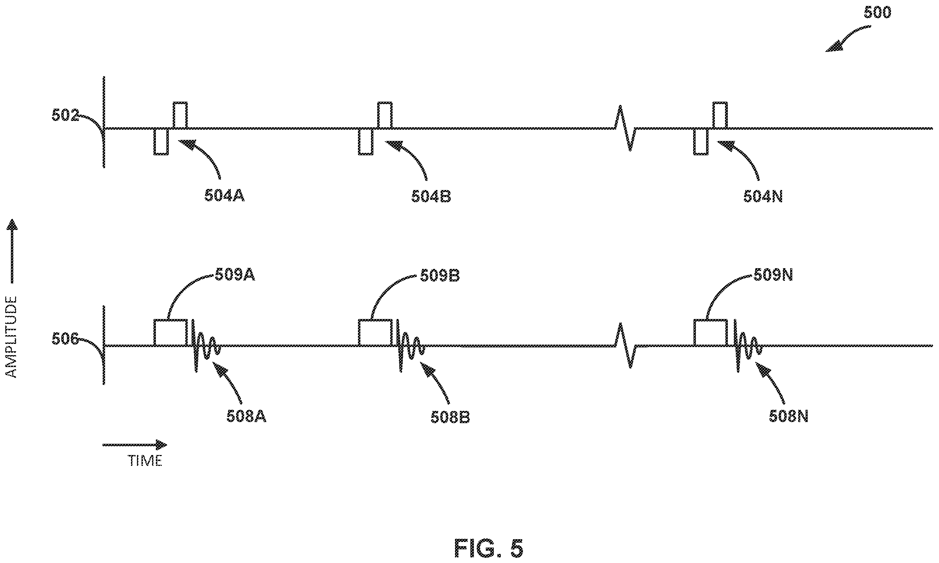

FIG. 5 is a timing diagram 500 illustrating one example of electrical stimulation pulses and respective sensed ECAPs, in accordance with one or more techniques of this disclosure. For convenience, FIG. 5 is described with reference to IMD 200 of FIG. 2. As illustrated, timing diagram 500 includes first channel 502, a plurality of control pulses 504A-504N (collectively "control pulses 504"), second channel 506, a plurality of respective ECAPs 508A-508N (collectively "ECAPs 508"), and a plurality of stimulation interference signals 509A-509N (collectively "stimulation interference signals 509"). In the example of FIG. 5, stimulation pulses 504 may or may not contribute to therapy for the patient. In any case, stimulation pulses 504 may elicit respective ECAPs 508 for the purpose of determining relative neural recruitment due to the stimulation pulses 504, which may be reflective as a growth curve specific to the posture state of the patient that was assumed with the ECAPs 508 were sensed.

First channel 502 is a time/voltage (and/or current) graph indicating the voltage (or current) of at least one electrode of electrodes 232, 234. In one example, the stimulation electrodes of first channel 502 may be located on the opposite side of the lead as the sensing electrodes of second channel 506. Stimulation pulses 504 may be electrical pulses delivered to the spinal cord of the patient by at least one of electrodes 232, 234, and stimulation pulses 504 may be balanced biphasic square pulses with an interphase interval. In other words, each of control pulses 504 are shown with a negative phase and a positive phase separated by an interphase interval. For example, a control pulse 504 may have a negative voltage for the same amount of time and amplitude that it has a positive voltage. It is noted that the negative voltage phase may be before or after the positive voltage phase. Stimulation pulses 504 may be delivered according to instructions stored in storage device 212 of IMD 200.

In some examples, each of stimulation pulses 504 may be a part of a sweep of pulses configured to determine a relationship between the stimulation parameter values of the pulses and the latency of one or more features of the resulting respective ECAPs 508. For example, the relationship may correspond to a latency curve of current amplitude values versus latency periods. In this manner, each of stimulation pulses 504 may differ from each other by a parameter value, such as an iteratively increasing current amplitude. In some examples, the sweep may also include iteratively decreasing current amplitude, or a separate sweep of iteratively decreasing current amplitude may be performed. Separate latency curves may be generated from the respective increasing and decreasing current amplitudes in order to adjust the sensing window based on whether or not the stimulation parameter value is increasing or decreasing from the previous stimulation pulse. In some examples, such sweeps may be performed for each posture state of a plurality of posture states in order to determine the latency curves or some characteristic related to ECAPs for that posture state. In one example, stimulation pulses 504 may have a pulse width of less than approximately 300 microseconds (e.g., the total time of the positive phase, the negative phase, and the interphase interval is less than 300 microseconds). In another example, control pulses 504 may have a pulse width of approximately 100 microseconds for each phase of the bi-phasic pulse. In some examples, the pulse width of stimulation pulses 504 may be longer than 300 microseconds, as long as the pulse width does not interfere with the detection of the desired one or more features of the elicited ECAPs 508. As illustrated in FIG. 5, stimulation pulses 504 may be delivered via channel 502. Delivery of stimulation pulses 504 may be delivered by leads 230 in a guarded cathode electrode combination. For example, if leads 230 are linear 8-electrode leads, a guarded cathode combination is a central cathodic electrode with anodic electrodes immediately adjacent to the cathodic electrode.

Second channel 506 is a time/voltage (and/or current) graph indicating the voltage (or current) of at least one electrode of electrodes 232, 234. In one example, the electrodes of second channel 506 may be located on the opposite side of the lead as the electrodes of first channel 502. ECAPs 508 may be sensed at electrodes 232, 234 from the spinal cord of the patient in response to stimulation pulses 504. ECAPs 508 are electrical signals which may propagate along a nerve away from the origination of stimulation pulses 504. In one example, ECAPs 508 are sensed by different electrodes than the electrodes used to deliver stimulation pulses 504. As illustrated in FIG. 5, ECAPs 508 may be recorded on second channel 506. In some examples, ECAPs 508 may not be sensed after each stimulation pulse 504.

Stimulation interference signals 509A, 509B, and 509N (e.g., the artifact of the stimulation pulses) may be sensed by leads 230 and may be sensed during the same period of time as the delivery of stimulation pulses 504. Since the interference signals may have a greater amplitude and intensity than ECAPs 508, any ECAPs arriving at IMD 200 during the occurrence of stimulation interference signals 509 may not be adequately sensed by sensing circuitry 206 of IMD 200. However, ECAPs 508 may be sufficiently sensed by sensing circuitry 206 because each ECAP 508, or at least a portion of ECAP 508 that includes one or more desired features of ECAP 508 that is used to detect the posture state and/or as feedback for stimulation pulses 504, falls after the completion of each a stimulation pulse 504. As illustrated in FIG. 5, stimulation interference signals 509 and ECAPs 508 may be recorded on channel 506.

In some examples, IMD 200, for example, may deliver the entire group of stimulation pulses 504 (e.g., a sweep) consecutively and without any other intervening pulses in order to detect ECAPs 508 from which respective characteristic values are determined. IMD 200 may then determine the relationship between the characteristic values from ECAPs 508 and the different parameter values of stimulation pulses 504. In one example, the sweep of pulses 504 may be delivered by IMD 200 during a break in delivery of other types of stimulation pulses.

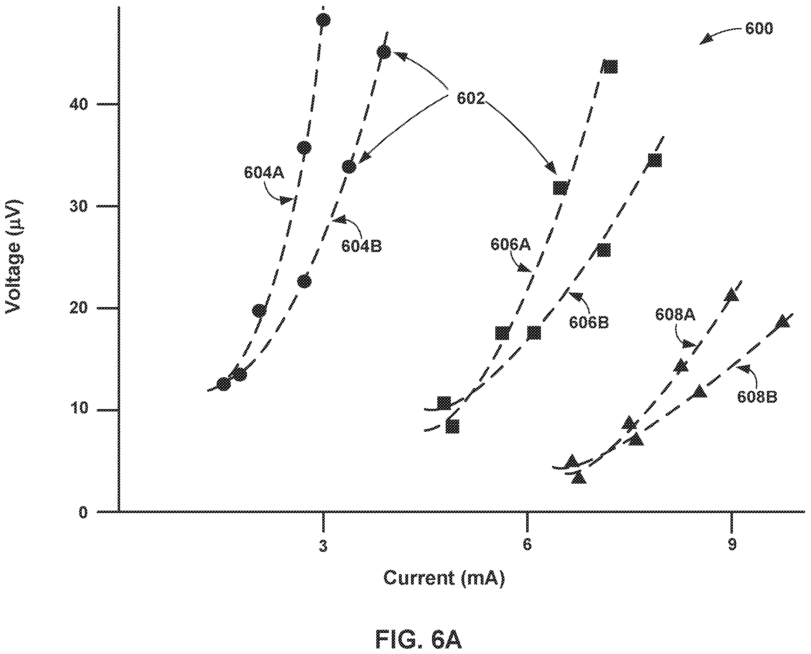

FIG. 6A is a graph 600 of example growth curves 604A, 604B, 606A, 606B, 608A, and 608B of sensed ECAPs from respective stimulation pulse amplitudes at respective posture states. Growth curves 604A and 604B (collectively "growth curves 604") may be associated with one posture state, growth curves 606A and 606B (collectively "growth curves 606") may be associated with a second posture state, and growth curves 608A and 608B (collectively "growth curves 608") may be associated with a third posture state Graph 600 illustrates example ECAPs shown as dots (growth curves 604), squares (growth curves 606), and triangles (growth curves 608) for respective different current amplitudes of stimulation pulses. ECAPs will sometimes not be generated until the stimulation pulse amplitude reaches a threshold, approximately at 4.5 mA current in the example of FIG. 6A. Then, as the current amplitude is increased, the ECAP amplitude also increases approximately linearly. This linear relationship is shown by growth curves 604, 606, 608. Besides growth curves varying based on the posture state of the patient, the slope may vary for each patient based on the type of electrodes implanted, where the electrodes are implanted, the sensitivity of the patient's neurons to stimulation, neurological dysfunction, or other factors. In addition, as discussed herein, the growth curves may vary according to whether the stimulation parameter for consecutive pulses was iteratively increased or iteratively decreased. The result is different growth curves for the same posture state, such as growth curves 604A and 604B.

While a patient is in a given posture state, sensed ECAPs may be detected for stimulation pulses with different current amplitudes. For example, each of growth curves 602, 604, and 606 may be for a single posture state, e.g., supine, prone, sitting, standing, or lying on the right side or left side. If a patient changes posture states, the growth curve can also change. When a patient changes posture states, e.g., supine to standing and standing to running, the corresponding growth curve can change as well. For example, growth curves 604 may be associated with a supine posture state, growth curves 606 may be associated with a sitting posture state, and growth curves 608 may be associated with a prone posture state. In some examples, a patient may change posture states, but the same growth curves, or gain values, may apply to the different posture state.

The slope of the growth curves 604, 606, and 608 that linearly increase may indicate the relationship between sensed ECAP amplitudes and pulse amplitudes. However, the different growth curves from each pair of growth curves 604, 606, and 608 are generated due to iteratively increasing or iteratively decreasing the current amplitude. For example, growth curve 604A may represent the relationship between ECAP characteristic values of ECAP voltage amplitude to increasing the current amplitude of consecutive pulses. Conversely, growth curve 604B may represent the relationship between ECAP characteristic values of ECAP voltage amplitude to decreasing the current amplitude of consecutive pulses. The steeper slope of growth curve 604A when compared to growth curve 604B indicates that increasing current amplitude between pulses causes a faster neural recruitment rate than when decreasing current amplitude between pulses. Therefore, a system may need to increase current amplitude at a slower rate (e.g., smaller incremental current amplitude increases) when increasing current amplitude than the rate used to decrease current amplitude. In this manner, each of growth curves 604A, 606A, and 608A may represent relationships between the ECAP characteristic value and iteratively increasing current amplitude, and each of growth curves 604B, 606B, and 608B may represent relationships between the ECAP characteristic value and iteratively decreasing current amplitude.

In some examples, the gain value used to increase or decrease stimulation parameter values may be inversely proportional to the slope of the growth curve of values of the characteristic of ECAP signals (e.g., an amplitude such as the N1-P2 amplitude or the amplitude of any peak of the ECAP signal) elicited from respective stimulation pulses delivered to the patient and at least partially defined by different values of a stimulation parameter (e.g., current amplitude, voltage amplitude, or pulse width). For example, the gain value for a patient may be used to dynamically adjust pulse amplitude based on the sensed ECAP amplitudes. In some examples, the gain may be approximated for a patient based on historical data for similar patients. In other examples, the system may generate a custom growth curve and gain specific to the patient before starting therapy with the system, such as using the calibration routine including sweeps of different pulses described herein. In some examples, IMD 110 may incorporate the growth curves for selection of gain in addition to determination of latency curves to determine the relationship between stimulation parameter values and sensing windows. In some examples, IMD 110 may utilize the sensing window selection process in order to accurately identify features in ECAP signals and generate accurate ECAP characteristic values needed to generate the growth curves described in FIG. 6A.

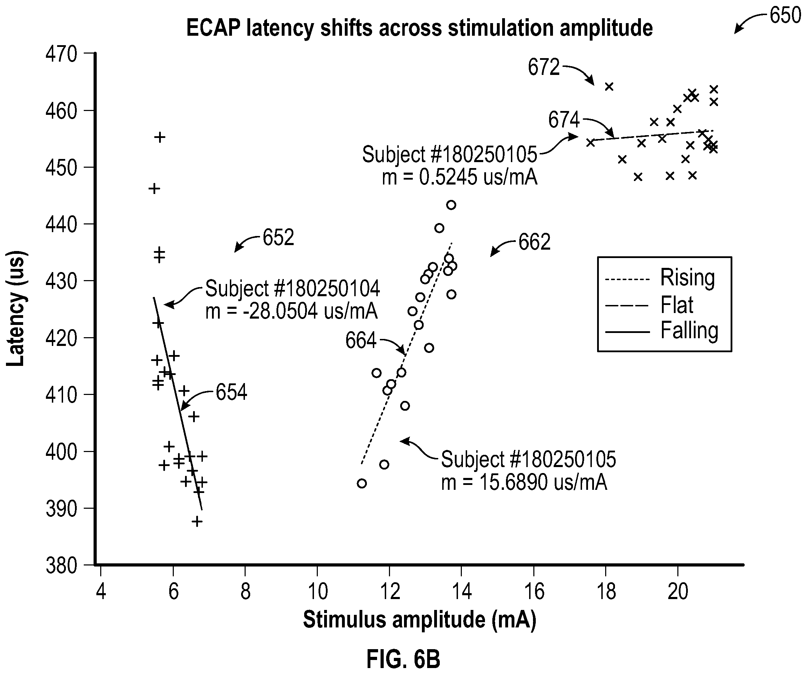

FIG. 6B is a graph 650 of example latency curves for different patients derived from sensed ECAPs elicited by stimulation pulses having different parameter values. As shown in FIG. 6B, stimulation parameter values for current amplitude are shown on the x-axis and latency is microseconds is shown in the y-axis. Each of the latency data 652, 662, and 672 correspond to different respective patients and indicate how latency changes for different stimulation parameters can be very patient-specific. Each of the data points (pluses for latency data 652, circles for latency data 662, and x's for latency data 672) were determined as part of the calibration sweep if stimulation pulses for that respective patient.

For latency data 652, latency curve 654 is a regression line fit to data points. Latency curve 654 is a falling curve. For latency data 662, latency curve 664 is a regression line fit to data points and shows a rising curve. For latency data 672, latency curve 674 is a regression line fit to data points and shown a generally flat curve (e.g., latency does not change significantly for different stimulation parameter values). The example data shown in graph 650 was generated based on the following example.

Fourteen subjects underwent conventional SCS trialing with percutaneous 1x8 leads placed near T9. At the end of their respective trial, the leads were connected to a custom device capable of stimulating and recording ECAP signals. ECAP signals were recorded as balanced, biphasic, 50 Hz stimulation was delivered across a range of postures and stimulation pulse widths. Stimulation amplitudes were increased incrementally up to a discomfort threshold and then decremented down to zero. Following filtering and interpolation, ECAP latencies are plotted in graph 650 as a function of the stimulation amplitude. Regression lines were fitted to these plots and arbitrarily classified by their slopes as "rising" (m > 12.5 µs/mA) "flat," (-12.5 ≤ m ≤ 12.5 µs/mA) or "falling" (m < -12.5 µs/mA). Further-to assess hysteresis effects in the DCs-intra-subject ECAP latency slope differences were compared with a t-test when incrementing the stimulation amplitude up to the DT versus decrementing down to zero.

Exemplary latencies as a function of stimulation amplitude are shown in graph 650 of FIG. 6B. In total, regression lines were fit to 112 separate recordings, and the slope classification results are shown in Table 1.

**Table 1: ECAP Slope Classification**

| ECAP Slope Classification | # of Recordings | # Exhibiting Significant Differences (p < 0.05) Incrementing vs. Decrementing Stim Amplitude |
|---|---|---|
| Rising (> 12.5 µs/mA) | 32/112 | 5/32 |
| Flat (-12.5 ≤ m ≤ 12.5 µs/mA) | 70/112 | 14/70 |
| Falling (< -12.5 µs/mA) | 10/112 | 3/10 |

ECAP latency shifts with stimulation amplitude changes were commonly observed. As indicated above, intra-subject hysteresis phenomena were noted where ECAP latency changes were also associated with the direction (incrementing versus decrementing) of stimulation amplitude change. Therefore, relationships between stimulation parameter values and latency may be established for both increasing and decreasing stimulation parameter values. The system may perform linear regressions on the patient data, which may require as few as two data points for latency and respective stimulation parameter values. However, additional data points may be collected to identify possible higher order relationships (e.g., non-linear relationships) for some patients and/or other stimulation parameter changes.

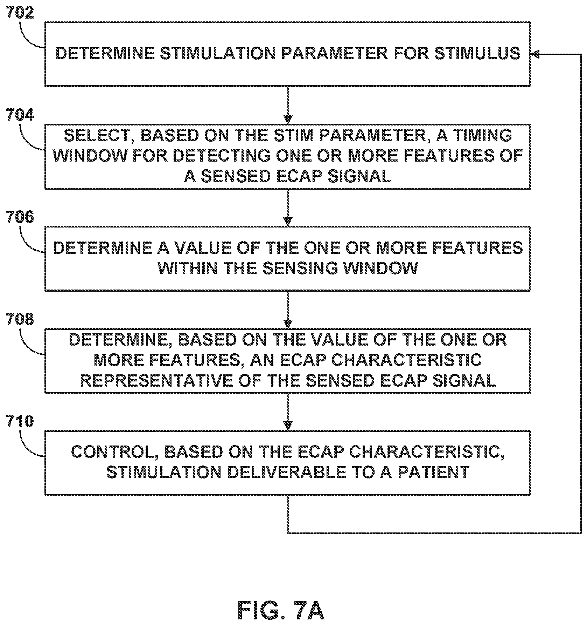

FIG. 7A is a flow diagram illustrating an example technique for selecting a sensing window for sensing an ECAP signal based on a stimulation parameter of a stimulation pulse that elicits the ECAP signal, in accordance with one or more techniques of this disclosure. For convenience, FIG. 7A is described with respect to IMD 200 of FIG. 2. However, the techniques of FIG. 7A may be performed by different components of IMD 200 or by additional or alternative medical devices. FIG. 7A will be described using stimulation pulses that eliciting detectable ECAP signals, where the pulses may or may not contribute to a therapeutic effect for the patient. IMD 200, for example, may select sensing windows to detect features of ECAP signals to determine ECAP characteristic values that may be used to adjust stimulation parameters. Although processing circuitry 208 will be described as performing much of the technique of FIG. 7A, other components of IMD 200 and/or other devices may perform some or all of the technique in other examples.

In the example operation of FIG. 7A, processing circuitry 208 determines the stimulation parameter value that will at least partially define the stimulus, or stimulation pulse, that is intended to elicit an ECAP signal (702). Although several stimulation parameters may define the stimulation pulse, such as current amplitude, pulse frequency, pulse width, electrode combination, etc., as few as one stimulation parameter may be needed for selecting the sensing window. For example, processing circuitry 208 may retrieve the value of the current amplitude for the stimulation pulse. Processing circuitry 208 then selects, based on the stimulation parameter, a timing window for detecting one or more features of the sensed ECAP signal (704). Selecting the sensing window may include selecting the duration of the sensing window and/or the delay or start time of the sensing window from delivery of the stimulation pulse. Processing circuitry 208 may receive the sensing window parameters from a look-up table that stores different sensing window parameters for one or more values or ranges of values for the stimulation parameter. In other examples, processing circuitry 208 may calculate one or more parameters of the sensing window using a function that defines a relationship between the stimulation parameter values and sensing window parameter(s).

Using the sensing window, processing circuitry 208 then determines a value of one or more features within the sensing window of the sensed ECAP signal elicited by the stimulation pulse (706). An example feature may be the N1 peak of the ECAP signal. In some examples, processing circuitry 208 may employ multiple sensing windows to detect respective features (e.g., peaks N1 and P2) of the ECAP signal used to determine an ECAP characteristic value (e.g., the absolute amplitude between the N1 and P2 peaks). Based on the value of the one or more features, processing circuitry 208 determines the ECAP characteristic representative of the sensed ECAP signal (708). Processing circuitry 208 can then control, based on the ECAP characteristic, electrical stimulation deliverable to the patient (710). Processing circuitry 208 may repeat the process of FIG. 7A for every sensed ECAP signal by returning to step 702. Alternatively, processing circuitry 208 may only perform this process any time the stimulation pulse stimulation parameter value has changed from the previous stimulation pulse used to elicit a previous ECAP signal.

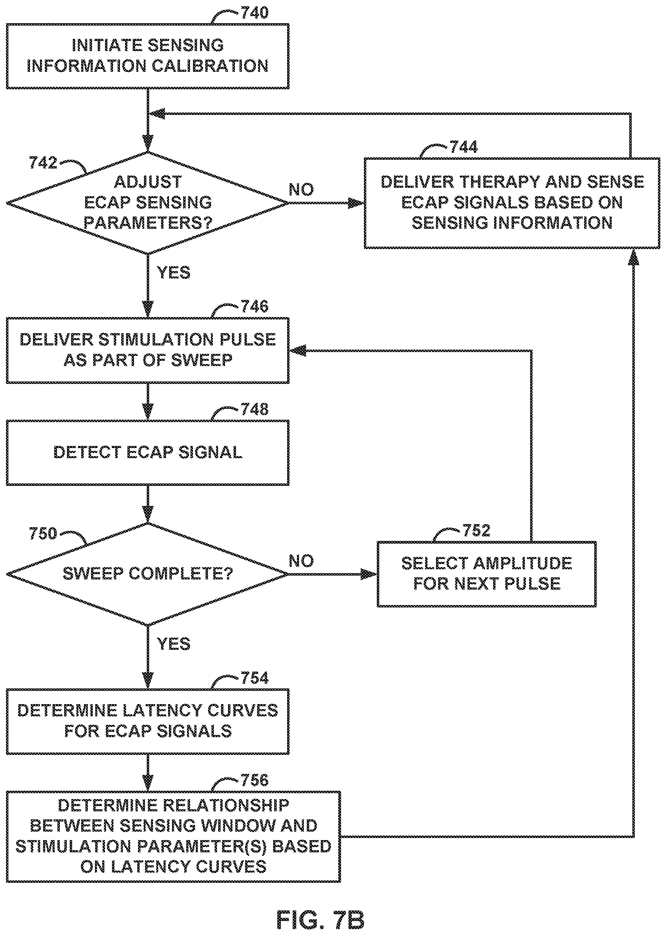

FIG. 7B is a flow diagram illustrating an example technique for determining a relationship between latency of ECAP features and stimulation parameter values, in accordance with one or more techniques of this disclosure. For convenience, FIG. 7B is described with respect to IMD 200 of FIG. 2. However, the techniques of FIG. 7B may be performed by different components of IMD 200 or by additional or alternative medical devices. FIG. 7B will be described using stimulation pulses that elicit detectable ECAP signals, where the pulses may or may not contribute to a therapeutic effect for the patient. IMD 200, for example, may use detected ECAP signals to determine latency curves from which a relationship between stimulation parameter values and sensing window parameters can be determined. Although processing circuitry 208 will be described as performing much of the technique of FIG. 7B, other components of IMD 200 and/or other devices may perform some or all of the technique in other examples.

In the example operation of FIG. 7B, processing circuitry 208 controls stimulation circuitry 202 to calibrate sensing information, such as determining latency curves for a patient and a relationship used to select sensing windows for ECAP signals (740). Processing circuitry 208 may then determine whether or not sensing parameters need to be determined or calibrated (742). If processing circuitry 208 does not have instructions to adjust ECAP sensing parameters, such as sensing window relationships ("NO" branch of block 742), processing circuitry 208 may continue to deliver stimulation and detect ECAP features according to the sensing parameters stored in memory (744). If processing circuitry 208 does have instructions to adjust ECAP sensing parameters, such as sensing window relationships ("YES" branch of block 742), processing circuitry 208 controls stimulation circuitry 202 to deliver the first stimulation pulse as part of a sweep of pulses with different parameter values (746). Processing circuitry 208 controls sensing circuitry 206 to detect the ECAP signal elicited by the stimulation pulse (748). If the sweep is not complete (e.g., there are more pulses of the sweep to be delivered) ("NO" branch of block 750), processing circuitry 208 selects the next stimulation parameter value (e.g., the next current amplitude) for the next stimulation pulse of the sweep (752) and controls stimulation circuitry 202 to deliver the next stimulation pulse of the sweep (746). A sweep of stimulation pulses may include at least two pulses, four or more pulses, or six or more pulses. In addition, the sweep may only increase the stimulation parameter value, only decrease the stimulation parameter value, or perform iterative increases in the stimulation parameter value and iterative decreases in the stimulation parameter value. Although more pulses may enable a more accurate relationship, as few pulses as possible may be used to reduce the amount of time needed to deliver pulses of the sweep and sense the resulting ECAP signals. In some examples, processing circuitry 208 may complete these sweeps for some or all posture states of the patient in order to determine relationships for each posture state.

If the sweep is complete and there are no more stimulation pulses of the sweep to be delivered ("YES" branch of block 750), processing circuitry 208 analyzes the detected ECAP signals from the sweep and determines one or more latency curves for these detected ECAP signals (754). The analysis of the detected ECAP signals may include determining the latency, or delay, or one or more features of the ECAP signal to the delivery of the stimulation pulse (e.g., the latency of the N1 peak or some other feature) and then associating that latency value to at least one parameter value (e.g., pulse current amplitude) that defined the stimulation pulse that elicited the characteristic value. All of the latency values and associated parameter values can be plotted, and processing circuitry 208 may determine a best fit line to the points and determine the slope of that best fit line for a particular latency curve. In other examples, processing circuitry 208 may determine a relationship between the latency value and respective parameter values that is different than a latency curve. In some examples, processing circuitry 208 may determine one latency curve for increasing the stimulation parameter value and another latency curve for decreasing the stimulation parameter value.

Processing circuitry 208 may then determine the relationship between one or more parameters of a sensing window and the stimulation parameter values based on the latency curves (756). For example, processing circuitry 208 may determine the start time of the sensing window and/or the duration of the sensing window for different stimulation parameter values such that the sensing window will capture the desired feature of the ECAP signal (e.g., an N1 or P2 peak). Processing circuitry 208 may store these relationships in memory 216. Processing circuitry 208 may then use the relationships as part of the sensing information for detecting features in subsequently detected ECAP signals (744). Processing circuitry 208 may perform the calibration process of FIG. 7B during initial set up and programming of IMD 200 and, in some examples, periodically during therapy as the patient's sensitivity to stimulation pulses may change over time. Processing circuitry 208 may request recalibration of the relationships for the sensing information in response to determining that ECAP characteristic values are not expected. Alternatively, a user may request, via a user interface, that processing circuitry 208 recalibrate the relationships for the sensing information if closed-loop control of stimulation therapy is no longer effective.

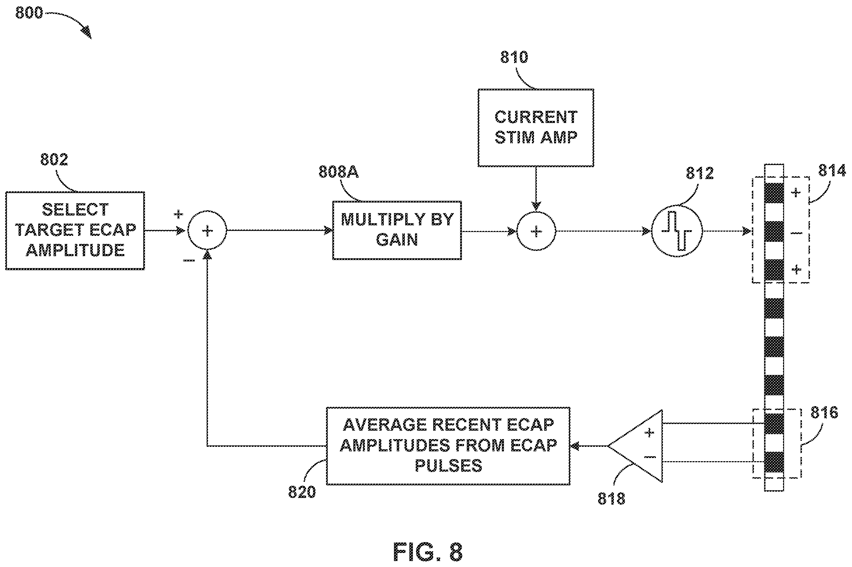

FIG. 8 is a diagram illustrating an example technique 800 for adjusting stimulation therapy. As shown in the example of FIG. 8, the system, such as IMD 200 or any other device or system described herein, may dynamically adjust pulse amplitude (or other parameter) based on the gain value representing the patient sensitivity to stimulation. Processing circuitry 208 of IMD 200 may control stimulation generation circuitry 204 to deliver a stimulation pulse to a patient. Processing circuitry 208 may then control sensing circuity 206 to sense an ECAP signal elicited by the pulse and then identify a characteristic of the ECAP signal (e.g., an amplitude of the ECAP signal). As described herein, processing circuitry 208 may determine one or more sensing windows for the ECAP signal based on a stimulation parameter value of the stimulation pulse that elicited the ECAP signal. Processing circuitry 208 may then control stimulation generation circuitry 204 to deliver the stimulation pulse according to the determined stimulation pulse.

As shown in the example of FIG. 8, a pulse 812 is delivered to the patient via electrode combination 814, shown as a guarded cathode of three electrodes. The resulting ECAP is sensed by the two electrodes at the opposing end of the lead of electrode combination 816 fed to a differential amplifier 818. For each sensed ECAP, processing circuitry 208 may measure an amplitude of a portion of the ECAP signal based one or more features within respective sensing windows, such as the N1-P2 voltage amplitude from the portion of the ECAP signal. Processing circuitry 208 may average the recently measured ECAP amplitudes, such as averaging the most recent, and consecutive, 2, 3, 4, 5, 6, or more ECAP amplitudes. In some examples, the average may be a mean or median value. In some examples, one or more ECAP amplitudes may be ignored from the calculations if the amplitude value is determined to be an error. The measured amplitude 820 (or average measured amplitude) is then subtracted from the selected target ECAP amplitude 802 to generate a differential amplitude (e.g., an ECAP differential value). The selected target ECAP amplitude 802 may be determined from an ECAP sensed when the physician or patient initially discovers effective therapy from the stimulation pulses. This target ECAP amplitude 802 may essentially represent a reference distance between the stimulation electrodes and the target neurons (e.g., the spinal cord for the case of SCS). The target ECAP amplitude 802 may also represent the target neural recruitment for the patient.

The differential amplitude may represent whether the stimulation intensity of the next stimulation pulse should increase or decrease in order to achieve the target ECAP amplitude 802. For example, a positive differential amplitude indicates that the measured amplitude (e.g., the determined characteristic value of the last one or more ECAP signals) is less than the target ECAP amplitude 802 and the stimulation intensity needs to increase in order to increase neural recruitment to achieve neural recruitment closer to the ECAP amplitude 802. Conversely, a negative differential amplitude indicates that the measured amplitude (e.g., the determined characteristic value of the last one or more ECAP signals) is greater than the target ECAP amplitude 802 and the stimulation intensity needs to decrease in order to decrease neural recruitment to achieve neural recruitment closer to the ECAP amplitude 802.

The differential amplitude is then multiplied by the gain value for the patient to generate a differential value 808A. Processing circuitry 208 may add the differential value 808A to the current pulse amplitude 810 to generate the new, or adjusted, pulse amplitude that at least partially defines the next pulse 812.

The following formulas may represent the function used to calculate the pulse amplitude of the next pulse 812. Equation 1 below represents an equation for calculating the new current amplitude using a linear function, wherein A_{C} is the current pulse amplitude, D is the differential amplitude by subtracting the measured amplitude from the target ECAP amplitude, G is a real number for the gain value, and A_{N} is the new pulse amplitude: ${A}_{N} = {A}_{C} + \left(D\times G\right)$ In some examples, the gain value G is a constant for increasing stimulation intensity or decreasing stimulation intensity. In this manner, the gain value G may not change for a given input. It is noted that different gain values may be employed for increasing stimulation than decreasing stimulation, as discussed herein. Alternatively, processing circuitry 208 may calculate the gain value G such that the gain value varies according to one or more inputs or factors. In this manner, for a given input or set of inputs, processing circuitry 208 may change the gain value G. Equation 2 below represents an example linear function for calculating the gain value, wherein M is a multiplier, D is the differential amplitude by subtracting the measured amplitude from the target ECAP amplitude, and G is the gain value: $G = M \times D$ Processing circuitry 208 may use the gain value G calculated in Equation 2 in Equation 1. This would result in Equation 1 being a non-linear function for determining the new current amplitude. According to Equation 2 above, the gain value G may be greater for larger differences between the measured amplitude and the target ECAP amplitude. Thus, gain value G will cause non-linear changes to the current amplitude. In this manner, the rate of change in the current amplitude will be higher for larger differences between the measured amplitude and the target ECAP amplitude and lower for smaller differences between the measured amplitude and the target ECAP amplitude. In other examples, a non-linear function may be used to calculate the gain value G.

The pulse width of the stimulation pulse may be greater than approximately 300 µs and less than approximately 1000 µs. In other examples, the pulse width of the stimulation pulse may be less than approximately 300 µs or greater than 1000 µs. The stimulation pulse may be a monophasic pulse followed a passive recharge phase. However, in other examples, the pulse may be a bi-phasic pulse that includes a positive phase and a negative phase. In some examples, a pulse may be less than 300 µs, but the following passive recharge phase or even an active recharge phase (of a bi-phasic pulse) may still obscure the detectable ECAP signal from that pulse. In other examples, the pulse width of the stimulation pulse may be greater than 300 µs, but some of the ECAP signal may be obscured by the stimulation pulse.

In some examples, depending upon, at least in part, pulse width of the stimulation pulse, IMD 110 may not sufficiently detect an ECAP signal because the stimulation pulse is also detected as an artifact that obscures the ECAP signal. If ECAPs are not adequately recorded, then ECAPs arriving at IMD 110 cannot be used to determine the efficacy of stimulation parameter settings, and electrical stimulation signals cannot be altered according to responsive ECAPs. In some examples, pulse widths may be less than approximately 300 µs, which may increase the amount of each ECAP signal that is detectable. Similarly, high pulse frequencies may interfere with IMD 110 sufficiently detecting ECAP signals. For example, at pulse frequency values (e.g., greater than 1 kHz) that cause IMD 110 to deliver another pulse before an ECAP from the previous pulse can be detected, IMD 110 may not be capable to detecting the ECAP.

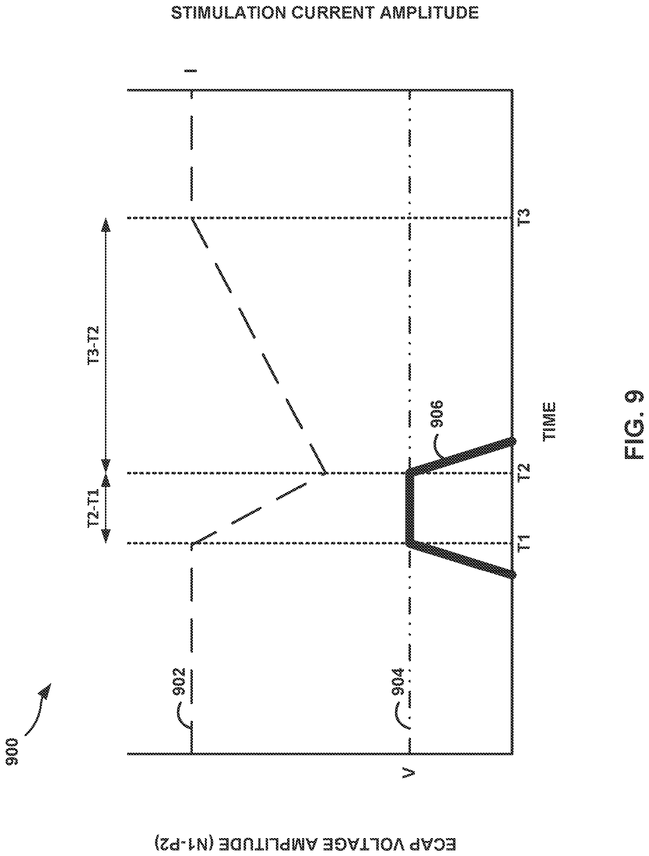

FIG. 9 illustrates a graph 900 that includes pulse current amplitude 902, threshold ECAP amplitude 904 (e.g., a type of threshold ECAP characteristic value), and sensed ECAP voltage amplitude 906 as a function of time, in accordance with one or more techniques of this disclosure. For convenience, FIG. 9 is described with respect to IMD 200 of FIG. 2. However, the techniques of FIG. 9 may be performed by different components of IMD 200 than as described herein or by additional or alternative medical devices.

Graph 900 illustrates a relationship between sensed ECAP voltage amplitude and stimulation pulse current amplitude. For example, pulse current amplitude 902 is plotted alongside ECAP voltage amplitude 906 as a function of time, showing how processing circuitry 208 can change stimulation current amplitude relative to ECAP voltage amplitude. In some examples, IMD 200 delivers a plurality of pulses at pulse current amplitude 902. Initially, IMD 200 may deliver a first set of stimulation pulses at current amplitude I. The first set of stimulation pulses may be delivered prior to time T1. In some examples, current amplitude I is less than 25 milliamps (mA) and can be between about 2 mA and about 18 mA. However, current amplitude I may be any current amplitude that IMD 200 can deliver to the patient and appropriate for effective stimulation therapy for the patient.

While delivering the first set pulses, IMD 200 may record ECAP voltage amplitude 906 from ECAPs elicited from the respective pulses. During transient patient movement, ECAP voltage amplitude 906 may increase if pulse current amplitude 902 is held constant and the distance between the electrodes and target nerve decreases. For example, as illustrated in FIG. 9, ECAP voltage amplitude 906 may increase prior to time T1 while stimulation current amplitude is held constant. An increasing ECAP voltage amplitude 906 may indicate that patient 102 is at risk of experiencing transient overstimulation due to the pulses delivered by IMD 200. To prevent patient 102 from experiencing transient overstimulation, IMD 200 may decrease pulse current amplitude 902 in response to ECAP voltage amplitude 906 exceeding the threshold ECAP amplitude 904. For example, if IMD 200 senses an ECAP having an ECAP voltage amplitude 906 meeting or exceeding threshold ECAP amplitude 904, as illustrated in FIG. 9 at time T1, IMD 200 may enter a decrement mode where pulse current amplitude 902 is decreased. In some examples, the threshold ECAP amplitude 904 is greater than 10 microvolts (µV) and less than 100 µV. For example, the threshold ECAP amplitude 904 can be 30 µV. In other examples, the threshold ECAP amplitude 904 is less than or equal to 10 µV or greater than or equal to 100 µV. The exact value of threshold ECAP amplitude 904 may depend on the patient's perception of the delivered stimulation, as well as the spacing between the sensing/stimulation electrodes and the neural tissue, whether or not stimulation intensity is increasing or decreasing, or other factors.

The decrement mode with a plurality of decrement rate settings may, in some cases, be stored in memory 216 of IMD 200 as a part of stimulation parameter settings 220. In the example illustrated in FIG. 9, the decrement mode is executed by IMD 200 over a second set of pulses which occur between time T1 and time T2. In some examples, to execute the decrement mode, IMD 200 decreases the pulse current amplitude 902 of each pulse of the second set of pulses according to a first linear function with respect to time. During a period of time in which IMD 200 is operating in the decrement mode (e.g., time interval T2-T1), ECAP voltage amplitude 906 of ECAPs sensed by IMD 200 may be greater than or equal to threshold ECAP amplitude 904.

In the example illustrated in FIG. 2, IMD 200 may sense an ECAP at time T2, where the ECAP has an ECAP voltage amplitude 906 that is less than threshold ECAP amplitude 904. The ECAP sensed at time T2 may, in some cases, be the first ECAP sensed by IMD 200 with a below-threshold amplitude since IMD 200 began the decrement mode at time T1. Based on sensing the ECAP at time T2, IMD 200 may deactivate the decrement mode and activate an increment mode. As discussed herein, IMD 200 may use a gain value selected for the increment mode such that the magnitude of the increase in stimulation parameter is appropriate for increasing the stimulation intensity of the next stimulation pulses. The increment mode with a plurality of increment rate settings may, in some cases, be stored in memory 216 of IMD 200 as a part of stimulation parameter settings 220. IMD 200 may execute the increment mode over a third set of pulses which occur between time T2 and time T3. In some examples, to execute the increment mode, IMD 200 increases the pulse current amplitude 902 of each pulse of the third set of pulses according to a second linear function with respect to time, back up to the initial current amplitude I that may be predetermined for therapy. In other words, IMD 200 increases each consecutive pulse of the third set of pulses proportionally to an amount of time elapsed since a previous pulse. Although IMD 200 may increase and decrease the amplitudes by linear functions in some examples, IMD 200 may employ non-linear functions in other examples. For example, the gain value may represent a non-linear function in which the increment or decrement changes exponentially or logarithmically according to the difference between the sensed ECAP characteristic value and the threshold ECAP amplitude 904.

When pulse current amplitude 902 returns to current amplitude I (e.g., the predetermined value for stimulation pulses), IMD 200 may deactivate the increment mode and deliver stimulation pulses at constant current amplitudes. By decreasing stimulation in response to ECAP amplitudes exceeding a threshold ECAP characteristic value and subsequently increasing stimulation in response to ECAP amplitudes falling below the threshold, IMD 200 may prevent patient 102 from experiencing transient overstimulation or decrease a severity and/or a time duration of transient overstimulation experienced by patient 102. In some examples, threshold ECAP amplitude 904 may include an upper threshold and a lower threshold, such that IMD 200 enters the decrement mode when the upper threshold is exceeded, IMD 200 enters the increment mode when the lower threshold is exceeded, and IMD 200 maintains stimulation parameter values when ECAP voltage amplitude 906 is between the upper threshold and the lower threshold.

The following examples are described herein. Example 1: A system includes processing circuitry configured to: determine a value of a stimulation parameter that at least partially defines an electrical stimulation pulse; select, based on the value of the stimulation parameter, a sensing window for detecting one or more features of a sensed evoked compound action potential (ECAP) signal elicited by the electrical stimulation pulse; determine a value of the one or more features within the sensing window from the sensed ECAP signal; and control, based on the value of the one or more features, subsequent electrical stimulation deliverable to a patient.

Example 2: The system of example 1, further comprising a memory storing a relationship between the stimulation parameter and the sensing window, wherein the processing circuitry is configured to select the sensing window based on the value of the stimulation parameter and the relationship stored in the memory.

Example 3: The system of example 2, wherein the relationship indicates one or more parameters defining the sensing window such that a first feature of the one or more features of the ECAP signal falls within the sensing window and a second feature of the ECAP signal does not fall within the sensing window.

Example 4: The system of any of examples 1 through 3, wherein the processing circuitry is configured to select the sensing window by at least determining a duration of the sensing window.

Example 5: The system of any of examples 1 through 4, wherein the processing circuitry is configured to select the sensing window by at least determining a start time of the sensing window based on delivery of the electrical stimulation pulse.

Example 6: The system of any of examples 1 through 5, wherein the one or more features of the ECAP signal comprises one of a P1, an N1, or a P2 peak of the ECAP signal.

Example 7: The system of any of examples 1 through 6, wherein the stimulation parameter comprises a current amplitude.

Example 8: The system of any of examples 1 through 7, wherein the stimulation parameter comprises a pulse width.

Example 9: The system of any of examples 1 through 8, wherein the processing circuitry is configured to determine, based on the value of the one or more features, an ECAP characteristic value representative of the sensed ECAP signal.

Example 10: The system of any of examples 1 through 9, further comprising stimulation circuitry configured to control the stimulation circuitry to deliver the plurality of electrical stimulation pulses as a sweep of pulses comprising iteratively increasing amplitude values of the stimulation parameter; determine a latency curve between the amplitude values of the stimulation parameter and a latency of the one or more features of respective ECAP signals elicited by respective electrical stimulation pulses of the plurality of electrical stimulation pulses; determine, based on the latency curve, a relationship between values of the stimulation parameter and one or more parameters of the sensing window; and control, based on the relationship and the first value of the stimulation parameter, the stimulation circuitry to deliver the subsequent electrical stimulation to the patient.

Example 11: The system of any of examples 1 through 10, further comprising an implantable medical device comprising the processing circuitry and stimulation circuitry configured to generate the electrical stimulation deliverable to the patient.

Example 12: A method comprising determining, by processing circuitry, a value of a stimulation parameter that at least partially defines an electrical stimulation pulse; selecting, by the processing circuitry and based on the value of the stimulation parameter, a sensing window for detecting one or more features of a sensed evoked compound action potential (ECAP) signal elicited by the electrical stimulation pulse; determining, by the processing circuitry, a value of the one or more features within the sensing window from the sensed ECAP signal; and controlling, by the processing circuitry and based on the value of the one or more features, subsequent electrical stimulation deliverable to a patient.

Example 13: The method of example 12, further comprising storing, in a memory, a relationship between the stimulation parameter and the sensing window, wherein selecting the sensing window comprises selecting the sensing window based on the value of the stimulation parameter and the relationship stored in the memory.

Example 14: The method of example 13, wherein the relationship indicates one or more parameters defining the sensing window such that a first feature of the one or more features of the ECAP signal falls within the sensing window and a second feature of the ECAP signal does not fall within the sensing window.

Example 15: The method of any of examples 12 through 14, wherein selecting the sensing window comprises determining a duration of the sensing window.

Example 16: The method of any of examples 12 through 15, wherein selecting the sensing window comprises determining a start time of the sensing window based on delivery of the electrical stimulation pulse.

Example 17: The method of any of examples 12 through 16, wherein the one or more features of the ECAP signal comprises one of a P1, an N1, or a P2 peak of the ECAP signal.

Example 18: The method of any of examples 12 through 17, wherein the stimulation parameter comprises a current amplitude.

Example 19: The method of any of examples 12 through 18, wherein the stimulation parameter comprises a pulse width.

Example 20: The method of any of examples 12 through 19, further comprising determining, based on the value of the one or more features, an ECAP characteristic value representative of the sensed ECAP signal.

Example 21: The method of any of examples 12 through 20, further comprising controlling stimulation circuitry to deliver a plurality of electrical stimulation pulses as a sweep of pulses comprising iteratively increasing amplitude values of the stimulation parameter; determining a latency curve between the amplitude values of the stimulation parameter and a latency of the one or more features of respective ECAP signals elicited by respective electrical stimulation pulses of the plurality of electrical stimulation pulses; determining, based on the latency curve, a relationship between values of the stimulation parameter and one or more parameters of the sensing window; and controlling, based on the relationship and the first value of the stimulation parameter, the stimulation circuitry to deliver the subsequent electrical stimulation to the patient.

Example 22: A computer-readable storage medium comprising instructions that, when executed by processing circuitry, causes the processing circuitry to: determine a value of a stimulation parameter that at least partially defines an electrical stimulation pulse; select, based on the value of the stimulation parameter, a sensing window for detecting one or more features of a sensed evoked compound action potential (ECAP) signal elicited by the electrical stimulation pulse; determine, a value of the one or more features within the sensing window from the sensed ECAP signal; and control, based on the value of the one or more features, subsequent electrical stimulation deliverable to a patient.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A system comprising processing circuitry (208) configured to:
determine a sensing window for sensing one or more features of an evoked compound action potential (ECAP) signal based on a value of a stimulation parameter that at least partially defined the stimulation pulse that elicited the ECAP signal.

2. The system of claim 1, wherein determine the sensing window includes determine a window duration.

3. The system of any of the preceding claims, wherein determine the sensing window includes determine a start of the sensing window after the stimulation pulse.

4. The system of any of the preceding claims, wherein the processing circuitry (208) is further configured to:
determine a value of each of the one or more features within the sensing window from the sensed ECAP signal; and
control, based on the value of each of the one or more features, subsequent electrical stimulation deliverable to a patient.

5. The system of any of the preceding claims, wherein the one or more features of the ECAP signal comprises one of a P1, an N1, or a P2 peak of the ECAP signal.

6. The system of any of the preceding claims, wherein the stimulation parameter comprises a current amplitude.

7. The system of any of the preceding claims, wherein the stimulation parameter comprises a pulse width.

8. The system of any of the preceding claims, wherein the processing circuitry (208) is configured to determine, based on the value of the one or more features, an ECAP characteristic value representative of the sensed ECAP signal.

9. The system of any of the preceding claims, further comprising stimulation circuitry (204) configured to generate the electrical stimulation deliverable to the patient.

10. The system of claim 9, wherein the stimulation circuitry is configured to deliver a plurality of electrical stimulation pulses at least partially defined by the stimulation parameter, wherein the processing circuitry (208) is configured to:
control the stimulation circuitry to deliver the plurality of electrical stimulation pulses as a sweep of pulses comprising iteratively increasing amplitude values of the stimulation parameter;
determine a latency curve between the amplitude values of the stimulation parameter and a latency of the one or more features of respective ECAP signals elicited by respective electrical stimulation pulses of the plurality of electrical stimulation pulses;
determine, based on the latency curve, a relationship between values of the stimulation parameter and one or more parameters of the sensing window; and
control, based on the relationship and the first value of the stimulation parameter, the stimulation circuitry to deliver the subsequent electrical stimulation to the patient.

11. The system of any of the preceding claims, further comprising an implantable medical device comprising the processing circuitry (208).

12. The system of any of the claims 1 to 10, further comprising an external programmer comprising the processing circuitry (208).

13. A computer-readable storage medium comprising instructions that, when executed by processing circuitry, causes the processing circuitry to:
determine a sensing window for sensing one or more features of an evoked compound action potential (ECAP) signal based on a value of a stimulation parameter that at least partially defined the stimulation pulse that elicited the ECAP signal.

14. The computer-readable storage medium of claim 13, wherein determine the sensing window includes determine a window duration.

15. The computer-readable storage medium of any of claim 13 or 14, wherein determine the sensing window includes determine a start of the sensing window after the stimulation pulse.

## Patentansprüche

1. System, umfassend eine Verarbeitungsschaltung (208), die konfiguriert ist zum:
Bestimmen eines Abtastungsfensters zum Abtasten eines oder mehrerer Merkmale eines Signals eines evozierten Summenaktionspotenzials (ECAP-Signal) basierend auf einem Wert eines Stimulationsparameters, der mindestens teilweise den Stimulationsimpuls definierte, der das ECAP-Signal auslöste.

2. System nach Anspruch 1, wobei das Bestimmen des Abtastungsfensters das Bestimmen einer Fensterdauer einschließt.

3. System nach einem der vorstehenden Ansprüche, wobei das Bestimmen des Abtastungsfensters das Bestimmen eines Starts des Abtastungsfensters nach dem Stimulationsimpuls einschließt.

4. System nach einem der vorstehenden Ansprüche,
wobei die Verarbeitungsschaltung (208) ferner konfiguriert ist zum:
Bestimmen eines Werts von jedem des einen oder der mehreren Merkmale innerhalb des Abtastungsfensters aus dem abgetasteten ECAP-Signal; und
Steuern, basierend auf dem Wert von jedem des einen oder der mehreren Merkmale, einer nachfolgenden elektrischen Stimulation, die an einen Patienten abgebbar ist.

5. System nach einem der vorstehenden Ansprüche, wobei das eine oder die mehreren Merkmale des ECAP-Signals eine von einer P1-, einer N1- oder einer P2-Spitze des ECAP-Signals umfasst.

6. System nach einem der vorstehenden Ansprüche, wobei der Stimulationsparameter eine Stromamplitude umfasst.

7. System nach einem der vorstehenden Ansprüche, wobei der Stimulationsparameter eine Impulsbreite umfasst.

8. System nach einem der vorstehenden Ansprüche, wobei die Verarbeitungsschaltung (208) konfiguriert ist, um basierend auf dem Wert des einen oder der mehreren Merkmale einen charakteristischen ECAP-Wert zu bestimmen, der repräsentativ für das abgetastete ECAP-Signal ist.

9. System nach einem der vorstehenden Ansprüche, ferner umfassend eine Stimulationsschaltung (204), die konfiguriert ist, um die an den Patienten abgebbare elektrische Stimulation zu generieren.

10. System nach Anspruch 9, wobei die Stimulationsschaltung konfiguriert ist, um eine Vielzahl von elektrischen Stimulationsimpulsen abzugeben, die mindestens teilweise durch den Stimulationsparameter definiert sind, wobei die Verarbeitungsschaltung (208) konfiguriert ist zum:
Steuern der Stimulationsschaltung, um die Mehrzahl von elektrischen Stimulationsimpulsen als einen Durchlauf von Impulsen abzugeben, umfassend die iterativ ansteigenden Amplitudenwerte des Stimulationsparameters;
Bestimmen einer Latenzkurve zwischen den Amplitudenwerten des Stimulationsparameters und einer Latenz des einen oder der mehreren Merkmale von jeweiligen ECAP-Signalen, die durch jeweilige elektrische Stimulationsimpulse der Mehrzahl von elektrischen Stimulationsimpulsen ausgelöst werden;
Bestimmen, basierend auf der Latenzkurve, einer Beziehung zwischen Werten des Stimulationsparameters und einem oder mehreren Parametern des Abtastungsfensters; und
Steuern, basierend auf der Beziehung und dem ersten Wert des Stimulationsparameters, der Stimulationsschaltung, um die nachfolgende elektrische Stimulation an den Patienten abzugeben.

11. System nach einem der vorstehenden Ansprüche, ferner umfassend eine implantierbare medizinische Vorrichtung, umfassend die Verarbeitungsschaltung (208).

12. System nach einem der Ansprüche 1 bis 10, ferner umfassend einen externen Programmierer, umfassend die Verarbeitungsschaltung (208).

13. Computerlesbares Speichermedium, umfassend Anweisungen, die, wenn sie durch die Verarbeitungsschaltung ausgeführt werden, die Verarbeitungsschaltung veranlasst wird zum:
Bestimmen eines Abtastungsfensters zum Abtasten eines oder mehrerer Merkmale eines Signals eines evozierten Summenaktionspotenzials (ECAP-Signal) basierend auf einem Wert eines Stimulationsparameters, der mindestens teilweise den Stimulationsimpuls definierte, der das ECAP-Signal auslöste.

14. Computerlesbare Speichermedium nach Anspruch 13, wobei das Bestimmen des Abtastungsfensters das Bestimmen einer Fensterdauer einschließt.

15. Computerlesbare Speichermedium nach Anspruch 13 oder 14, wobei das Bestimmen des Abtastungsfensters das Bestimmen eines Starts des Abtastungsfensters nach dem Stimulationsimpuls einschließt.

## Revendications

1. Système comprenant une circuiterie de traitement (208) configurée pour :
déterminer une fenêtre de détection pour détecter une ou plusieurs caractéristiques d'un signal de potentiel d'action composite évoqué (ECAP) sur la base d'une valeur d'un paramètre de stimulation qui a défini au moins partiellement l'impulsion de stimulation qui a déclenché le signal ECAP.

2. Système selon la revendication 1, dans lequel la détermination de la fenêtre de détection comporte la détermination d'une durée de fenêtre.

3. Système selon l'une quelconque des revendications précédentes, dans lequel la détermination de la fenêtre de détection comporte la détermination d'un début de la fenêtre de détection après l'impulsion de stimulation.

4. Système selon l'une quelconque des revendications précédentes,
dans lequel la circuiterie de traitement (208) est en outre configurée pour :
déterminer une valeur de chaque caractéristique parmi la ou les caractéristiques à l'intérieur de la fenêtre de détection à partir du signal ECAP détecté ; et
commander, en fonction de la valeur de chaque caractéristique parmi la ou les caractéristiques, une stimulation électrique ultérieure pouvant être administrée à un patient.

5. Système selon l'une quelconque des revendications précédentes, dans lequel la ou les caractéristiques du signal ECAP comprennent l'un parmi un pic P1, un pic N1 ou un pic P2 du signal ECAP.

6. Système selon l'une quelconque des revendications précédentes, dans lequel le paramètre de stimulation comprend une amplitude de courant.

7. Système selon l'une quelconque des revendications précédentes, dans lequel le paramètre de stimulation comprend une largeur d'impulsion.

8. Système selon l'une quelconque des revendications précédentes, dans lequel la circuiterie de traitement (208) est configurée pour déterminer, en fonction de la valeur de la ou des caractéristiques, une valeur caractéristique ECAP représentant le signal ECAP détecté.

9. Système selon l'une quelconque des revendications précédentes, comprenant en outre une circuiterie de stimulation (204) configurée pour générer la stimulation électrique pouvant être administrée au patient.

10. Système selon la revendication 9, dans lequel la circuiterie de stimulation est configurée pour administrer une pluralité d'impulsions de stimulation électrique au moins partiellement définies par le paramètre de stimulation, dans lequel la circuiterie de traitement (208) est configurée pour :
commander le circuit de stimulation pour administrer la pluralité d'impulsions de stimulation électrique en tant que balayage d'impulsions comprenant des valeurs d'amplitude croissante de manière itérative du paramètre de stimulation ;
déterminer une courbe de latence entre les valeurs d'amplitude du paramètre de stimulation et une latence de la ou des caractéristiques des signaux ECAP respectifs déclenchés par des impulsions de stimulation électrique respectives de la pluralité d'impulsions de stimulation électrique ;
déterminer, en fonction de la courbe de latence, une relation entre des valeurs du paramètre de stimulation et un ou plusieurs paramètres de la fenêtre de détection ; et
commander, en fonction de la relation et de la première valeur du paramètre de stimulation, le circuit de stimulation pour administrer la stimulation électrique ultérieure au patient.

11. Système selon l'une quelconque des revendications précédentes, comprenant en outre un dispositif médical implantable comprenant la circuiterie de traitement (208).

12. Système selon l'une quelconque des revendications 1 à 10, comprenant en outre un programmeur externe comprenant la circuiterie de traitement (208).

13. Support de stockage lisible par ordinateur comprenant des instructions qui, lorsqu'elles sont exécutées par une circuiterie de traitement, amènent la circuiterie de traitement à :
déterminer une fenêtre de détection pour détecter une ou plusieurs caractéristiques d'un signal de potentiel d'action composite évoqué (ECAP) sur la base d'une valeur d'un paramètre de stimulation qui a défini au moins partiellement l'impulsion de stimulation qui a déclenché le signal ECAP.

14. Support de stockage lisible par ordinateur selon la revendication 13, dans lequel la détermination de la fenêtre de détection comporte la détermination d'une durée de fenêtre.

15. Support de stockage lisible par ordinateur selon l'une quelconque des revendications 13 ou 14, dans lequel la détermination de la fenêtre de détection comporte la détermination d'un début de la fenêtre de détection après l'impulsion de stimulation.
